(19) Europäisches Patentamt
European Patent Office
Office européen des brevets

(11) Veröffentlichungsnummer: **0 354 418 B1**

(12) **EUROPÄISCHE PATENTSCHRIFT**

(45) Veröffentlichungstag der Patentschrift: **18.05.94**

(21) Anmeldenummer: **89113917.2**

(22) Anmeldetag: **28.07.89**

Die Akte enthält technische Angaben, die nach dem Eingang der Anmeldung eingereicht wurden und die nicht in dieser Patentschrift enthalten sind.

(51) Int. Cl.5: **C07D 405/06**, C07D 407/06, C07D 409/06, C07D 207/337, C07D 213/48, C07D 213/55, C07D 237/08, C07D 239/26, C07D 307/54, C07D 309/30, C07D 333/24, //C07D207/333, C07D333/22,C07D307/46, A61K31/365

# (54) 6-Fluor-3,5-dihydroxycarbonsäuren und deren Derivate, Verfahren zu ihrer Herstellung, ihre Verwendung als Arzneimittel, pharmazeutische Präparate und Zwischenprodukte.

(30) Priorität: **06.08.88 DE 3826814**

(43) Veröffentlichungstag der Anmeldung:
**14.02.90 Patentblatt 90/07**

(45) Bekanntmachung des Hinweises auf die Patenterteilung:
**18.05.94 Patentblatt 94/20**

(84) Benannte Vertragsstaaten:
**AT BE CH DE ES FR GB GR IT LI LU NL SE**

(56) Entgegenhaltungen:
**EP-A- 0 221 025** **EP-A- 0 306 929**
**EP-A- 0 307 342** **DE-A- 3 722 806**
**DE-A- 3 722 807** **DE-A- 3 722 809**
**DE-A- 3 800 785** **US-A- 4 681 893**

(73) Patentinhaber: **HOECHST AKTIENGESELLSCHAFT**

**D-65926 Frankfurt(DE)**

(72) Erfinder: **Beck, Gerhard, Dr.**
**Gustav-Freytag-Strasse 24**
**D-6000 Frankfurt am Main(DE)**
Erfinder: **Bartmann, Wilhelm, Prof. Dr.**
**Am Dachsbau 5**
**D-6232 Bad Soden am Taunus(DE)**
Erfinder: **Wess, Günther, Dr.**
**Langenselbolder Weg 35**
**D-6455 Erlensee(DE)**
Erfinder: **Granzer, Ernold, Dr. Dr.**
**Falkensteiner Strasse 24**
**D-6233 Kelkheim (Taunus)(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 354 418 B1

JOURNAL OF MEDICINAL CHEMISTRY, Band 28, Nr. 3, 1985, Seiten 347-358; G.E. STOKKER et al.:"3-hydroxy-3-methylglutaryl-coenzyme A reductase inhibitors. 1. Structural modification of 5-substituted 3,5-dihydroxypentanoic acids and their lactone derivatives"

JOURNAL OF MEDICINAL CHEMISTRY, Band 29, Nr.2, 1986, Seiten 170-181; G.E. STOKKER et al.:"3-Hydroxy-3-methylglutaryl-coenzyme A reductase inhibitors. 3. 7-(3,5-disubstituted [1,1'-biphenyl]-2-yl)-3,5-dihydroxy-6-heptenoic acids and their lactone derivatives"

TETRAHEDRON, Band 40, Nr. 15, 1984, Seiten 2871-2878, US; F. CAMPS et al.:"Synthesis of dienic fluorinated analogs of insect sex pheromones"

TETRAHEDRON, Band 35, 1979, Seiten 2645-2653, GB; C. CHUIT et al. :" Réaction du diméthylcuprate de lithium avec les dérivés carbonylés alpha,beta-insaturés alpha-fluorés et alpha-chlorés"

CHEMICAL AND PHARMACEUTICAL BULLETIN, Band 35, Nr. 4, 1987, Seiten 1666-1669; T. TAGUCHI et al.: "Synthesis of 5-fluoroarachidonic acid and its biotransformation to 5-fluoro-12-hydroxyeicosatetraenoic acid"

**Beschreibung**

Das Enzym 3-Hydroxy-3-methylglutaryl-Coenzym-A-Reduktase (HMG-CoA-Reduktase) katalysiert die Bildung von Mevalonsäure aus 3-Hydroxy-3-methylglutaryl-Coenzym A (HMG-CoA). Diese Reaktion spielt eine zentrale Rolle bei der Biosynthese des Cholesterins. Derivate der 3-Hydroxy-3-methyl-glutarsäure (HMG) und der Mevalonsäure sind als Hemmer der Cholesterinbiosynthese beschrieben worden (M. R. Boots et al., J. Pharm. Sci. 69, 506 (1980), F. M. Singer et al., Proc. Soc. Exper. Biol. Med. 102, 370 (1959), H. Feres, Tetrahedron Lett. 24, 3769 (1983)). 3-Hydroxy-3-methyl-glutarsäure selbst zeigt an der Ratte und im Humanversuch signifikante cholesterinsenkende Wirkung (Z. Beg, Experientia 23, 380 (1967), ibid 24, 15 (1968), P. J. Lupien et al., Lancet 1978, 2, 283).

Es wurde nun gefunden, daß die Fluor-dihydroxycarbonsäuren der allgemeinen Formel I sowie die entsprechenden Laktone der Formel II Hemmstoffe der HMG-CoA-Reduktase sind.

Die Erfindung betrifft daher 6-Fluor-3,5-dihydroxycarbonsäuren und deren Derivate der allgemeinen Formel I

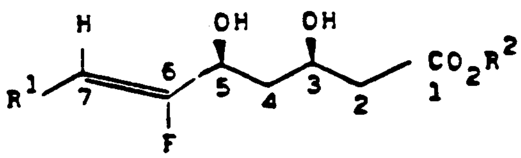

I

und die entsprechenden Laktone der Formel II

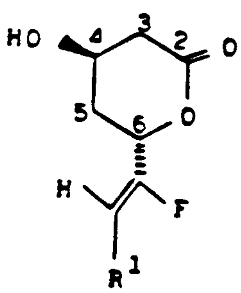

II

In den allgemeinen Formeln I und II bedeuten

$R^1$ die Gruppe der substituierten 6-Ring-Heteroaromaten b

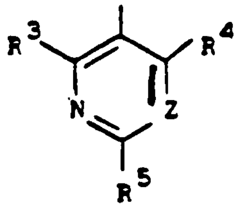

b

worin

Z einen Rest der Formel -CH oder ein Stickstoffatom

$R^3$, $R^4$, $R^5$ unabhängig voneinander Wasserstoff,
einen geradkettigen oder verzweigten Alkyl- oder Alkenylrest mit jeweils bis zu 6 C-Atomen, der gegebenenfalls am terminalen Kohlenstoff durch einen Cycloalkyl- oder Cycloalkenylrest mit jeweils 3-6 C-Atomen substituiert sein kann, einen cyclischen gesättigten oder bis zu zweifach ungesättigten Kohlenwasserstoffrest mit 3-7 C-Atomen

oder einen aromatischen Rest ausgewählt aus der Gruppe Phenyl, Furyl, Thienyl, Pyridinyl, der gegebenenfalls im Kern 1-3 gleiche oder verschiedene Substituenten der folgenden Gruppe tragen kann: Halogen, Trifluormethyl, Alkyl oder Alkenyl mit je bis zu 6 C-Atomen, Hydroxy, Alkoxy mit 1-6 C-Atomen, Carboxy, Carbalkoxy mit 1-6 C-Atomen im Alkoxyteil, bedeuten und

$R^2$ Wasserstoff, einen geradkettigen oder verzweigten Alkyl- oder Alkenylrest mit bis zu 8 C-Atomen, einen Benzylrest, dessen Kern 1-2 fach mit Halogen oder einem Alkylrest mit 1-4 C-Atomen substituiert sein kann, Alkalimetall oder ein Ammoniumion $NR^{13}R^{14}R^{15}R^{16}$, wobei $R^{13}$, $R^{14}$, $R^{15}$ und $R^{16}$ gleich oder verschieden sind und Wasserstoff, Alkyl mit 1-4 C-Atomen oder Hydroxyalkyl mit 1-4 C-Atomen bedeutet.

Die Erfindung betrifft die reinen Enantiomeren mit der in der allgemeinen Formel I angegebenen absoluten Konfiguration 4R, 6S bzw. der in Formel II wiedergegebenen absoluten Konfiguration 3R, 5S.

Unter den Substituenten $R^3$ und $R^4$ sind bevorzugt ein geradkettiger oder verzweigter Alkylrest mit 1-4 C-Atomen, ein Cycloalkylrest mit 5-6 C-Atomen,
ein Cycloalkylmethyl- oder Cycloalkenylmethylrest mit einer Ringgröße von 5-6 C-Atomen,
ein Phenylrest, der gegebenenfalls 1-3 gleiche oder verschiedene Substituenten der folgenden Gruppe tragen kann:
Halogen, Trifluormethyl, Alkyl mit 1-4 C-Atomen,
Hydroxy, Alkoxy mit 1-4 C-Atomen oder Carbalkoxy mit 1-4 C-Atomen im Alkoxyteil.

Unter den Bedeutungen für $R^5$ sind bevorzugt Wasserstoff, ein geradkettiger oder verzweigter Alkyl- oder Alkenylrest mit bis zu 6 C-Atomen,
ein Cycloalkyl- oder Cycloalkenylrest mit je 5-6 C-Atomen, ein Phenyl- oder Pyridinylrest, wobei die aromatischen Reste gegebenenfalls 1-3 gleiche oder verschiedene Substituenten der folgenden Gruppen tragen können:
Halogen, Alkyl mit 1-4 C-Atomen, Hydroxy, Alkoxy mit 1-4 C-Atomen oder Carbalkoxy mit 1-4 C-Atomen im Alkoxyteil.

Unter den Substituenten $R^3$ sind besonders bevorzugt:
Methyl, Isopropyl, sek.-Butyl, tert.-Butyl, Cyclohexyl, Phenyl, 4-Chlorphenyl, 4-Fluorphenyl, 4-Methoxyphenyl, 4-Fluor-3-methylphenyl, 3,5-Dimethylphenyl, Cyclohexylmethyl, 4-Trifluormethylphenyl.

Unter den Substituenten $R^4$ sind besonders bevorzugt:
Methyl, Isopropyl, sek.-Butyl, tert.-Butyl, Cyclohexyl, Phenyl, 4-Chlorphenyl, 4-Fluorphenyl, 4-Methoxyphenyl, 4-Fluor-3-methylphenyl, 3,5-Dimethylphenyl, Cyclohexylmethyl, 4-Trifluormethylphenyl.

Unter den Substituenten $R^5$ sind besonders bevorzugt:
Wasserstoff, Methyl, Isopropyl, tert.-Butyl, Cyclohexyl, Phenyl, 4-Fluorphenyl, 2,5-Dimethylphenyl, 3,5-Dimethylphenyl, 4-Trifluormethylphenyl.

Die Erfindung betrifft ferner ein Verfahren zur Herstellung der Verbindungen der allgemeinen Formeln I und II, das dadurch gekennzeichnet ist, daß man

a) entsprechend substituierte Aldehyde der Formel III

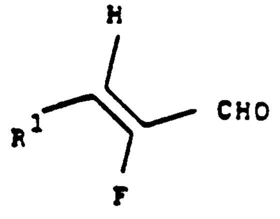

III

worin $R^1$ die angegebene Bedeutung hat, in die entsprechenden Hydroxyester der allgemeinen Formel IV überführt.

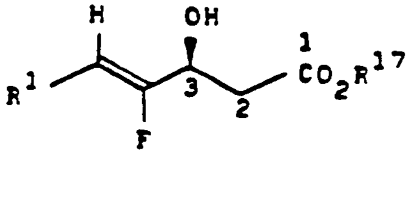

IV

worin $R^1$ die angegebene Bedeutung hat und $R^{17}$ eine geeignete optisch aktive Säureschutzgruppe, die die Stereochemie an 3-C bestimmt, darstellt oder einen Alkylrest mit 1-8 C-Atomen

b) die optisch aktiven Verbindungen der Formel IV mit achiralen Essigsäureesterenolaten entweder direkt oder nach vorheriger Überführung in die entsprechenden Alkylester, vorzugsweise Methylester, in die optisch aktiven Verbindungen der Formel V überführt,

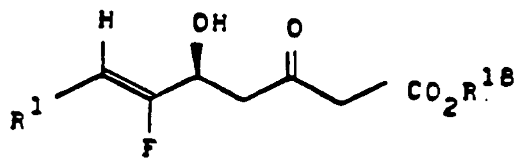

V

worin $R^1$ die angegebene Bedeutung hat und $R^{18}$ Alkyl mit 1-8 C-Atomen ist,

c) die Hydroxyketoester der Formel V in die entsprechenden 6-Fluor-3,5-dihydroxyverbindungen der Formel I

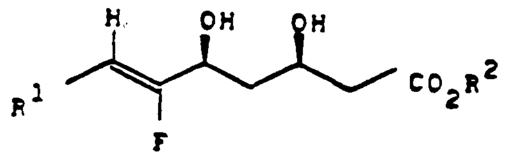

I

worin $R^1$ die zu Formel I angegebene Bedeutung hat, und $R^2$ Alkyl mit 1-8 C-Atomen ist, überführt und eine erhaltene Verbindung gegebenenfalls verseift zu einer Verbindung der Formel I, worin $R^2$ ein Metallkation darstellt, daraus gegebenenfalls die freie Säure ($R^2$ = Wasserstoff) in Freiheit setzt und die freie Säure gegebenenfalls in Verbindungen der Formel I mit $R^2$ = Alkyl- oder Alkenyl mit bis zu 8 C-Atomen, Ammoniumion, Benzyl oder entsprechend substituiertes Benzyl überführt,

d) und eine erhaltene Verbindung der Formel I gegebenenfalls in ein Lakton der Formel II überführt

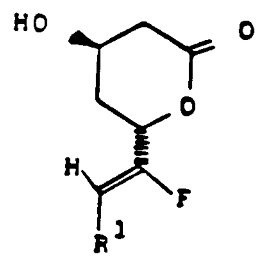

II

worin $R^1$ die angegebene Bedeutung hat.

Die Umwandlung von Verbindungen der Formel III über Verbindungen der Formel IV in Verbindungen der Formel V erfolgt je nach Gegebenheiten und Erfordernissen nach verschiedenen Varianten, wie z.B.

1. Umsetzung der Enolate von achiralen Essigsäureestern, wie z.B. Ethyl oder Propylestern, die mit starken Basen, vorzugsweise LDA, in THF bereitet werden, mit Aldehyden der Formel III führt in Lösungsmitteln wie beispielsweise THF bei Temperaturen zwischen -78 °C und 0 °C zu racemischen Verbindungen der Formel IV, worin $R^{17}$ eine achirale Säureschutzgruppe, wie z.B. die Ethyl- oder Propylgruppe, bedeutet. Umsetzung mit einem weiteren Essigsäureesterenolat führt in Lösungsmitteln wie beispielsweise THF bei -78 °C bis Raumtemperatur zu racemischen Verbindungen der Formel V.

2. Umsetzung von Aldehyden der Formel III mit Lithium-, Natrium-, Kalium- oder Magnesiumenolaten von optisch aktiven Essigsäureestern führt in Lösungsmitteln, wie THF bei -78 °C bis 0 °C zu optisch aktiven Verbindungen der Formel IV. In diesem Fall bedeutet $R^{17}$ eine geeignete optisch aktive Säureschutzgruppe, die die Stereochemie an 3-C bestimmt. Vorzugsweise wird hier die Gruppe

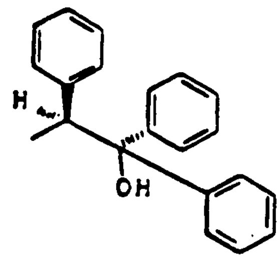

verwendet, die nach M. Braun und R. Devant (Tetrahedron Lett. 25, 5031 (1984)) die 3 R Konfiguration ergibt und aus L-(+)-Mandelsäure hergestellt wird. Es eignen sich aber auch andere chirale optisch aktive Gruppen. Die nun optisch aktiven Verbindungen der Formel IV werden mit achiralen Essigsäureesterenolaten nach Variante 1. entweder direkt in die jetzt optisch aktiven Verbindungen der Formel V überführt oder nach vorheriger Überführung in die entsprechenden Alkylester, vorzugsweise Methylester.

Die Umwandlung von Verbindungen der Formel V in Verbindungen der Formel I erfolgt z.B. in Analogie zu einem literaturbekannten Verfahren (K. Narasaka und H.C. Pai, Chemistry Lett. 1980 1415). Zunächst wird mit einem Trialkylboran, vorzugsweise Triethylboran, in THF bei Raumtemperatur umgesetzt und dann bei -78°C bis 0°C mit Natriumborhydrid, gegebenenfalls unter Zusatz von Methanol reduziert. Auf diese Weise erhält man die in Formel I angegebenen stereochemischen Verhältnisse (syn. 3,5-dihydroxy).

Die über Variante 1. erhaltenen Verbindungen der Formeln I und II können ggf. nach den bekannten Verfahren der Racematspaltung in die reinen Enantiomeren getrennt werden. Die Salze und Säuren der Verbindungen der allgemeinen Formel I werden nach allgemein bekannten Methoden erhalten.

Die Laktone der Formel II werden ebenfalls nach bekannten Verfahren erhalten beispielsweise durch Wasserabspaltung aus den offenen Dihydroxycarbonsäuren der Formel I, $R^2$ gleich H, in Benzol, Hexan oder Toluol unter Zusatz von p-Toluolsulfonsäure oder Trifluoressigsäure bei Raumtemperatur bis Rückflußtemperatur.

Die Herstellung der Aldehyde der Formel III kann aus den Verbindungen der allgemeinen Formel VI

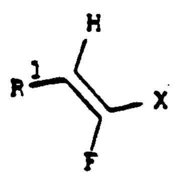 VI (X = $-COOR^{19}$, $-C{\equiv}N$)

wobei X eine Nitrilgruppe oder eine Estergruppe ist, auf unterschiedliche Weise erfolgen:

a) Die Reaktion der Nitrile der Formel VI (X = $-C{\equiv}N$), worin $R^1$ die zu Formel I angegebene Bedeutung hat, mit Diisobutylaluminiumhydrid (Dibah) in THF bei -10°C bis 50°C führt nach Hydrolyse direkt zu den Aldehyden der Formel III.

b) Die Reaktion der Carbonsäureester der Formel VI (X = $-COOR^{19}$), worin $R^1$ die zu Formel I angegebene Bedeutung hat und $R^{19}$ Alkyl mit 1-8 C-Atomen ist, führt durch Reduktion mit Dibah in THF bei -10°C bis 50°C zunächst zu den entsprechenden Alkoholen der Formel VII,

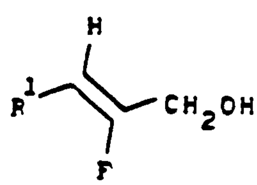 VII

worin $R^1$ die zu Formel I angegebenen Bedeutung hat. Durch Oxydation mit üblichen Oxydationsmitteln wie Chrom (VI)oxid, Swern Reagenz (Oxalylchlorid/DMSO/$NEt_3$), $CrO_3$ • Pyr, Mangandioxid, oder nach K.B. Sharpless et al., Tetrahedron Lett. 29, 2503 (1976) mit N-Methylmorpholin/$(PPh_3)_3$ $RuCl_2$ in üblichen Lösungsmitteln wie z.B. $CH_2Cl_2$, Aceton können die Alkohole VII bei Temperaturen zwischen -50°C und

+30 °C in die Aldehyde der Formel III überführt werden.

Die Verbindungen der Formel VI (X = $-COOR^{19}$, $-C{\equiv}N$) werden aus den Aldehyden der Formel VIII,

$R^1-CH=O$ VIII

worin $R^1$ die zu Formel I angegebene Bedeutung hat, gewonnen, und zwar durch Umsetzung mit den Phosphonaten der allgemeinen Formel IX

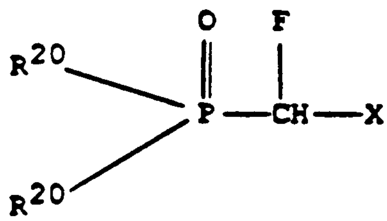

IX

worin $R^{20}$ eine Alkoxygruppe mit 1-6 C-Atomen oder eine Phenylgruppe, die 1 bis 3-fach substituiert sein kann mit Halogen oder Alkoxy mit 1-5 C-Atomen, bedeutet, und X die zu Formel VI angegebene Bedeutung hat, nach Horner-Emmons-Wittig (Horner et al. Chem. Ber. 91, 61 (1958)).

Die Umsetzung erfolgt vorzugsweise in Lösungsmitteln wie Dimethoxyäthan, THF in Gegenwart einer Base wie z.B. Natriumhydrid oder BuLi bei Temperaturen zwischen -20 °C und Raumtemperatur. Es fallen überwiegend die E-Isomeren an, die ggf. durch Kristallisation oder Chromatographie gereinigt werden können.

Die Phosphonate der Formel IX werden nach literaturbekannten Verfahren erhalten, z.B. nach G. Etemad-Moghadam, J. Seyden-Penne, Bull. Soc. France 3, 448 (1985) oder EP-A-224.417, oder H. Machleidt, R. Wessendorf, Liebigs Ann. Chem. 674, 1 (1964).

Die Aldehyde der Formel VIII werden durch Oxydation mit üblichen Oxydationsmitteln wie z.B. Chrom (VI)oxid, Swern Reagenz, $CrO_3$ • Pyr. in üblichen Lösungsmitteln wie z.B. $CH_2Cl_2$, Aceton etc. bei Temperaturen zwischen -50 °C und +30 °C aus den Alkoholen der allg. Formel X

$R^1-CH_2OH$ X

worin $R^1$ die zu Formel I angegebene Bedeutung hat, erhalten. Die Herstellung des Alkohols der allg. Formel X erfolgt nach in der Literatur beschriebenen Verfahren wie aus der nachfolgenden Zusammenstellung erkennbar ist. Entsprechend den Literaturstellen (3), (8) und (9) erhält man direkt die Aldehyde VIII.

Herstellung der Alkohole X

| $R^1$ | $R^1-CH_2OH$ X |
|---|---|
| – 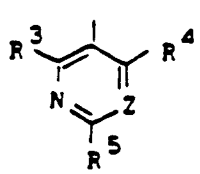 | Deutsche Offenlegungsschrift 38 23 045 (1) (entspr. EP-A-0307342; US-Patentanmeldung Serial-No.: 216 458) |

Die Reinigung von Zwischenprodukten erfolgt falls notwendig durch Destillation, Kristallisation, Flash-Chromatographie oder HPLC.

Außer den in den Beispielen beschriebenen Verbindungen lassen sich nach dem erfindungsgemäßen Verfahren die folgenden Verbindungen herstellen:

Z-6(S)-(2-(4,6-Bis-(4-Fluorphenyl)-2-(1-methylethyl)-pyridin-3-yl)-1-fluorethenyl)-4-(R)-hydroxy-3,4,5,6-tetrahydro-2H-pyran-2-on

Z-6(S)-(1-Fluor-2-(4-(4-fluorphenyl)-2-(1-methylethyl)-6-phenylpyridin-3-yl)-ethenyl)-4-(R)-hydroxy-3,4,5,6-tetrahydro-2H-pyran-2-on

6Z-7-(4,6-Bis-(4-fluorphenyl)-2-(1-methylethyl)-pyridin-3-yl)-3R, 5S-dihydroxy-6-fluorhept-6-ensäure-t-butylester

6Z-3(R), 5(S)-Dihydroxy-6-fluor-7-(4-(4-fluorphenyl)-2-(1-methylethyl)-6-phenylpyridin-3-yl)-hept-6-ensäure-t-butylester

Biologische Testsysteme:

1. HMG-CoA-Reduktase-Aktivität in Enzympräparationen

Die HMG-CoA-Reduktase-Aktivität wurde an solubilisierten Enzympräparationen aus Lebermikrosomen von Ratten gemessen, die nach Umstellung im Tag-Nacht-Rhythmus mit Cholestyramin ($^R$Cuemid) induziert wurden.

Als Substrat diente (S,R) $^{14}$C-HMG-CoA, die Konzentration von NADPH wurde während der Inkubation durch ein regenerierendes System aufrechterhalten. Die Abtrennung von $^{14}$C-Mevalonat vom Substrat und anderen Produkten (z.B. $^{14}$C-HMG) erfolgte über Säulenelution, wobei das Elutionsprofil jeder Einzelprobe ermittelt wurde.

Auf die ständige Mitführung von $^3$H-Mevalonat wurde verzichtet, weil es sich bei der Bestimmung um die Relativangabe der Hemmwirkung handelt. In einer Versuchsreihe wurden jeweils die enzymfreie Kontrolle, der enzymhaltige Normalansatz (= 100 %) und solche mit Präparatezusätzen, Endkonzentration $10^{-5}$ bis $10^{-9}$ M, zusammen behandelt. Jeder Einzelwert wurde als Mittelwert aus 3 Parallelproben gebildet. Die Signifikanz der Mittelwertsunterschiede zwischen präparatefreien und präparatehaltigen Proben wurde nach dem t-Test beurteilt.

Nach der obenbeschriebenen Methode wurden von den erfindungsgemäßen Verbindungen z.B. folgende Hemmwerte auf die HMG-CoA-Reduktase ermittelt [$IC_{50}$/mol/Liter bedeutet die molare Konzentration der Verbindung pro Liter, die für eine 50 %ige Hemmung erforderlich ist]:

Tabelle 1

| Verbindung gem. Bsp. | $IC_{50}$/mol/Liter |
|---|---|
| 10 a | $2,9 \times 10^{-9}$ |
| 10 b | $9,0 \times 10^{-9}$ |
| 10 c | $1,8 \times 10^{-9}$ |

2. Supprimierung bzw. Inhibierung der HMG-CoA-Reduktase in Zellkulturen von HEP-G2-Zellen

Monolayers von HEP-G2-Zellen in lipoproteinfreiem Nährmedium wurden mit entsprechenden Konzentrationen der Prüfsubstanzen eine bestimmte Zeit (z.B. 1 Stunde) vorinkubiert, nach Zugabe des markierten Präkursors, z.B. $^{14}$C-Natriumacetat, wurde die Inkubation fortgesetzt (z.B. 3 Stunden). Nach Zugabe eines internen Standards ($^3$H-Cholesterin) wurde ein Teil der Zellen alkalisch verseift. Die Lipide der verseiften Zellen wurden mit Chloroform/Methanol extrahiert. Dieses Lipidgemisch wurde nach Zusatz von Träger-Cholesterin präparativ dünnschichtchromatographisch getrennt, die Cholesterinbande nach dem Sichtbarmachen mit Jod-Dampf isoliert und die aus dem $^{14}$C-Präkursor gebildete Menge $^{14}$-C-Cholesterin szintigraphisch bestimmt. In einem aliquoten Teil der Zellen wurde Zellprotein bestimmt, so daß die in der Zeiteinheit pro mg Zellprotein gebildete Menge $^{14}$C-Cholesterin berechnet werden kann. Durch Vergleich dieses Wertes mit der Menge an $^{14}$C-Cholesterin, die pro mg Zellprotein und Zeiteinheit in einer gleichbehandelten, jedoch prüfsubstanzfreien Kultur gebildet wurde, ergab sich die Hemmwirkung des jeweiligen Prüfpräparates auf die Cholesterin-Biosynthese von HEP-G2-Zellkulturen.

**Prüfung von Substanzen auf Hemmung der Cholesterin-Biosynthese in konfluenten Zellkulturen (Monolayer) von HEP-G2-Zellen**

1. lipoproteinfreies Medium (DMEM) — 24 h
2. Inkubation mit Prüfpräparaten — 1 h
3. Inkubation mit $^{14}C$-Acetat — 3 h
4. Cytolyse
5. DC-Trennung des Reaktionsproduktes $^{14}C$-Cholesterin
6. Isolierung des $^{14}C$-Cholesterin
7. Szintillationsmessung

Interner Standard $^{3}H$-Cholesterin

Zellprotein-bestimmung

8. **Ergebnis in nmol $^{14}C$-Cholesterin/mg Zellprotein im Vergleich zur Lösungsmittelkontrolle**

Nach der obenbeschriebenen Methode wurden von den erfindungsgemäßen Verbindungen z.B. folgende Hemmwerte der Cholesterinbiosynthese (in HEP-G2-Zellen) ermittelt (der $IC_{50}$/mo Liter ist diejenige Konzentration der Verbindung, die eine 50 %ige Hemmung der Cholesterinbiosynthese bewirkt) (Tab. 2).

Tabelle 2

| Verbindung gem. Bsp. | $IC_{50}$/mol/Liter |
|---|---|
| 10 a | $1{,}9 \times 10^{-8}$ |
| 11 a | $1{,}8 \times 10^{-8}$ |
| 10 c | $2{,}5 \times 10^{-9}$ |
| 10 b | $4{,}6 \times 10^{-9}$ |
| 11 b | $2{,}0 \times 10^{-8}$ |

Die Verbindungen der allgemeinen Formel I bzw. II zeichnen sich aus durch starke Hemmung der HMG-CoA-Reduktase, des geschwindigkeitsbestimmenden Enzyms der Cholesterinbiosynthese.

Das durch $IC_{50}$-Werte im Bereich von $10^{-7}$-$10^{-9}$ mol pro Liter charakterisierte Ausmaß der Hemmung ist bei Verbindungen der allgemeinen Formel I bzw. II deutlich höher als bei literaturbekannten, vollsynthetischen HMG-CoA-Reduktase-Inhibitoren wie z.B. den von G. E. Stokker et al., J. Med. Chem. 29, 170 (1986) beschriebenen.

Das Enzym HMG-CoA-Reduktase ist in der Natur weit verbreitet. Es katalysiert die Bildung von Mevalonsäure aus HMG-CoA. Diese Reaktion ist ein zentraler Schritt der Cholesterinbiosynthese (vgl. J. R.

Sabine in CRC Series in Enzyme Biology: 3-Hydroxy-3-methylglutaryl Coenzyme A Reductase, CRC Press Inc. Boca Raten, Florida 1983 (ISBN 0-8493-6551-1)).

Hohe Cholesterinspiegel werden mit einer Reihe von Erkrankungen, wie z.B. koronarer Herzkrankheiten oder Arteriosklerose in Verbindung gebracht. Daher ist die Senkung erhöhter Cholesterinspiegel zur Vorbeugung und Behandlung solcher Erkrankungen ein therapeutisches Ziel.

Ein Ansatzpunkt dazu liegt in der Hemmung bzw. Verminderung der endogenen Cholesterinbiosynthese. Hemmstoffe der HMG-CoA-Reduktase blockieren die Cholesterinbiosynthese auf einer frühen Stufe.

Die Verbindungen der allgemeinen Formel I bzw. II eignen sich daher als Hypolipidemika und zur Behandlung bzw. Prophylaxe arteriosklerotischer Veränderungen.

Die Erfindung betrifft daher auch pharmazeutische Präparate auf Basis dieser Verbindungen sowie ihre Verwendung als Arzneimittel, insbesondere als Hypolipidemika und zur Prophylaxe arteriosklerotischer Veränderungen.

Die Anwendung der Verbindungen der Formel I bzw. II als Hypolipidemika oder Anti-arteriosklerotika erfolgt in oralen Dosen von 3 bis 2500 mg pro Tag, vorzugsweise jedoch im Dosisbereich von 10 - 500 mg. Diese Tagesdosen können nach Bedarf auch in zwei bis vier Einzeldosen aufgeteilt werden oder in Retardform verabreicht werden. Das Dosierungsschema kann vom Typ, Alter, Gewicht, Geschlecht und medizinischem Zustand des Patienten abhängen.

Ein zusätzlicher cholesterinsenkender Effekt läßt sich durch gleichzeitige Gabe der erfindungsgemäßen Verbindungen mit gallensäurebindenden Stoffen, wie z.B. Anionenaustauscherharzen, erzielen. Die Gallensäureausscheidung führt zu einer verstärkten Neusynthese und damit zu einem erhöhten Cholesterinabbau (vgl. M. S. Brown, P. T. Koranen und J. C. Goldstein, Science 212, 628 (1981); M. S. Brown, J. C. Goldstein, Spektrum der Wissenschaft 1985, 1, 96).

Die erfindungsgemäßen Verbindungen der Formel I können in Form der Ester, als freie Säuren oder in Form ihrer physiologischen unbedenklichen anorganischen oder organischen Salze zur Anwendung kommen. Die Verbindungen der Formel I bzw. II können in Form ihrer wäßrigen Lösungen oder Suspensionen oder auch gelöst oder suspendiert in pharmakologisch unbedenklichen organischen Lösungsmitteln wie ein- oder mehrwertigen Alkoholen wie z.B. Ethanol, Ethylenglykol oder Glycerin, in Triacetin, in Alkohol-Acetaldehyddiacetalgemischen, Ölen wie z.B. Sonnenblumenöl oder Lebertran, Ethern, wie z.B. Diethylenglykoldimethylether, oder auch Polyethern, wie z.B. Polyethylenglykol, oder auch in Gegenwart anderer pharmakologisch unbedenklicher Polymerträger wie z.B. Polyvinylpyrrolidon oder in festen Zubereitungen zur Anwendung gelangen.

Für die Verbindung der Formel I bzw. II sind feste, oral verabreichbare Zubereitungsformen bevorzugt, die die üblichen Hilfsstoffe enthalten können. Sie werden nach üblichen Methoden hergestellt.

Als Zubereitung für die orale Anwendung eignen sich insbesondere Tabletten, Dragees oder Kapseln. Eine Dosierungseinheit enthält vorzugsweise 10 bis 500 mg Wirkstoff.

Die Verbindungen der Formel III, IV, V, VI und VII sind neu und stellen wertvolle Zwischenprodukte für die Herstellung von Verbindungen der Formel I dar. Die Erfindung betrifft daher auch diese Verbindungen sowie Verfahren zu ihrer Herstellung.

Vorbemerkung: NMR-Spektren wurden, sofern nichts anderes angegeben, in $CDCl_3$ mit internem Standard TMS gemessen. Zur Klassifizierung von NMR-Signalen gelten folgende Abkürzungen: S = Singulett, brs = breites Singulett, d = Dublett, t = Triplett, q = Quartett, h = Heptett, mc = zentriertes Multiplett, m = Multiplett.

Schmelzpunkte sind unkorriergiert.

Es gelten folgende Substituentenabkürzungen:

i = iso, t = tertiär, c = cyclo.

Beispiel 1

Allgemeine Vorschrift zur Herstellung von Verbindungen der allgemeinen Formel VIII

Beispiel 1a

4,6-Bis(4-Fluorphenyl)-2-(1-methylethyl)-pyrimidin-3-aldehyd

12 g (0,12 Mol) $CrO_3$ werden in 400 ml abs. $CH_2Cl_2$ unter Argon unter Rühren aufgeschlämmt. Nach 15 Min. wird auf 0 °C abgekühlt und eine Lösung von 19,4 ml (0,24 Mol) Pyridin in 100 ml abs. $CH_2Cl_2$ zugetropft. Anschließend läßt man unter Rühren auf Raumtemperatur erwärmen und tropft 8 g (24 mMol) 3-Hydroxymethyl-4,6-bis-(4-fluorphenyl)-2-(1-methylethyl)-pyrimidin (Formel X, hergestellt wie in der deutschen Patentanmeldung P 38 23 045.3 beschrieben) zu. Es wird 2 Std. bei Raumtemperatur gerührt. Nach beendeter Reaktion wird die Methylenchloridphase abdekantiert, i. Vak. eingeengt und der Rückstand über Flashchromatographie ($CH_2Cl_2$) an Kieselgel gereinigt.

Ausbeute: 7,2 g (89 % d. Th.) helle Kristalle Fp. 119-122 °C VIIIa
$R_f$ = 0,84 (Cyclohexan/Essigester = 2 : 1).
$^1$H-NMR: δ-Werte in ppm 1,4 (d, 6H, $CH_3$), 4,0 (h, 1H, CH), 7,0-7,9 (m, 6H, aromat. Prot.), 8,5-8,9 (m, 2H, aromat. Prot.), 10,1 (s, 1H, CH=O)
MS: m/e = 338 $C_{20}H_{16}F_2N_2O$

Beispiel 1b - 1d

Die Verbindungen VIII b-VIII d wurden in analoger Weise wie in Beispiel 1a beschrieben dargestellt (vgl. Tab. 1).

Tabelle I

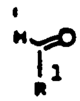 VIII

| Beispiel | Verbindung | $R^1$ | Ausbeute % | $R_f$-Wert Fp°C | $^1H$-NMR: δ/ppm = MS : m/e = |
|---|---|---|---|---|---|
| 1 a | VIIIa | 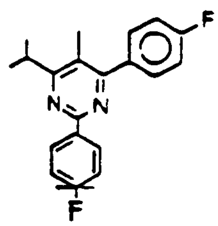 | 89 % | vgl. Beschreibung Beispiel 1a | |
| b | VIIIb | 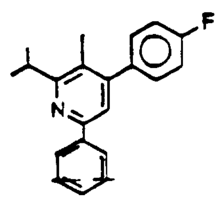 | 82.5 % | Fp. 104° | $C_{21}H_{18}FNO$ (319) |
| c | VIIIc | 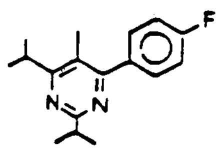 | 73 % | Fp. 61°C | $C_{17}H_{17}FN_2O$ (284) |
| d | VIII d | 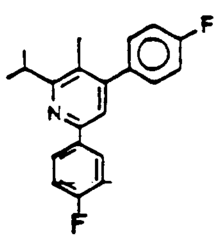 | 69 % | helles Öl | $C_{21}H_{17}F_2NO$ (337) |

Beispiel 2

Allgemeine Vorschrift zur Herstellung von Verbindungen der allgemeinen Formel VI ($X = CO_2R^{19}$)

Beispiel 2a

2Z-2-Fluor-3[4,6-bis-(4-fluorphenyl)-2-(1-methylethyl)-pyrimidin-3-yl]-acrylsäureäthylester

8,06 g (0,022 Mol) Ethyl-phosphinoxy-fluoracetat (Formel IX, $R^{20} = C_6H_5$, $X = CO_2Et$; hergestellt wie von G. Etemad-Moghadam u. J. Seyden-Penne in Bull. Soc. Chim. France 3, 448 - 454 (1985) beschrieben) werden in 180 ml abs. THF unter Argon gelöst. Zu dieser Lösung wird unter Rühren bei 0°C 15 ml (24

mMol) einer Lösung von Buthyllithium in Hexan zugetropft. Nach 30 Min. Rühren wird bei Raumtemperatur 7,6 g (22,4 mMol) 4,6-Bis- (4-fluorphenyl)-2-(1-methylethyl)-pyrimidin-3-aldehyd (aus Bsp. 1a) in 10 ml abs. THF gelöst zugetropft. Nach ca. 3 Std. ist die Reaktion beendet. Es wird mit 200 ml Diathyläther versetzt und 2 x mit gesättigter Natriumchloridlösung extrahiert. Die org. Phase wird abgetrennt, mit $MgSO_4$ getrocknet, filtriert und i. Vak. eingeengt.

Ausbeute: 7,8 g (75 % d. Th.) helle Kristalle Fp. 85-89 °C VIa $R_f$ = 0,52 (Cyclohexan/Essigester = 10 : 1)

$^1$H-NMR: δ-Werte in ppm 1,35 (t, 3H, $CH_3$), 1,40 (d, 6H, $CH_3$), 3,2 (m, 1H, CH), 4,4 (q, 2H, $OCH_2CH_3$), 7,12 (d, J = 33Hz, 1H, CH = CF-), 7,1-7,2 (m, 4H, aromat Prot.), 7,7 und 8,6 (m, 2+2H, aromat Prot.)

MS: m/e = 426 $C_{24}H_{21}F_3N_2O_2$

Beispiel 2a-2d

Die Verbindungen VI b-VId wurden in analoger Weise wie in Beispiel 2a beschrieben dargestellt (vgl. Tab. 2)

Beispiel 3

Allgemeine Vorschrift zur Herstellung von Verbindungen der allgemeinen Formel VI (X = CN)

Beispiel 3a

2-Fluor-3[4,6-bis-(4-fluorphenyl)-2-(1-methylethyl)-pyrimidin-3-yl]-acrylsäurenitril

0,28 g (6 mMol) Natriumhydrid 55 %ige Suspension in Öl werden in 20 ml DME abs. aufgeschlämmt. Unter Rühren wird 1,17 g (0,95 ml, 6 mMol) 2-(O,O-Diethylphosphono)-2-fluoracetonitril (Formel IX, $R^{20}$ = $OC_2H_5$, X = C ≡ N; hergestellt wie in EP-A-224 417 beschrieben) zugetropft und 15 Min. gerührt. Dann werden 1,6 g (5 mMol) 4,6-Bis-(4-Fluorphenyl)-2-(1-methylethyl)-pyrimidin-3-aldehyd aus Bsp. 1a zugetropft. Nach 1 Std. wird mit 2 N Salzsäure auf pH 7 gestellt und das Reaktionsgemisch i. Vak. eingeengt. Der Rückstand wird mit 200 ml EE/$H_2O$ im Verhältnis 1 : 1 versetzt und die Essigesterphase abgetrennt, 2 x mit gesättigter Natriumchloridlösung gewaschen, getrocknet, filtriert und i. Vak. eingeengt und der Rückstand auf einer Kieselgelsäule gereinigt (Cyclohexan/Essigester = 10 : 1)

Ausbeute: 1,6 g (72 % d. Th.) gelbe Kristalle Fp. 112 °C VIa', X = CN
$R_f$ = 0,49 (Z-Isomer) Cyclohexan/Essigester = 10:1

$^1$H-NMR: δ-Werte in ppm 1,4 (d, 6H, $CH_3$), 3,18 (m, 1H, CH), 6,78 (d, 1H, J = 32 Hz, CH = CF), 7,2-8,5 (m, 8H, aromat. Prot.)

MS: m/e = 361 $C_{22}H_{17}F_2N_3$

Beispiel 3b-3d

Die Verbindungen VI b'-VI d' (X = CN) können in analoger Weise, wie in Beispiel 3a beschrieben dargestellt werden.

Tabelle 2

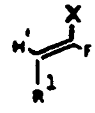 VI (X = $CO_2Et$)

| Bei-spiel | Verbin-dung | $R^1$ | Ausbeute % | $R_f$-Wert (Z) Fp°C (Z) | $^1$H-NMR: δ/ppm = MS: m/e = |
|---|---|---|---|---|---|
| 2 a | VIa | 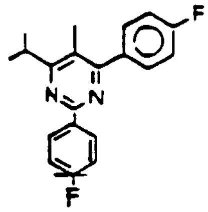 | 75 % | Fp. 89° vgl. Beschreibung Beispiel 2a | |
| 2 b | VIb | 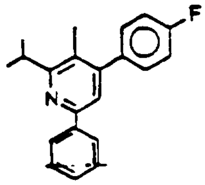 | 81 % | $R_f$ = 0.55 | $C_{25}H_{23}NF_2O_2$ (407) |
| 2 c | VI c | 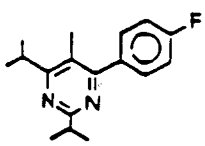 | 71 % | $R_f$ = 0.57 | $C_{21}H_{22}N_2F_2O_2$ (372) |
| 2 d | VI d | 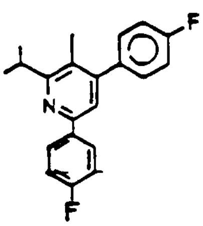 | 69 % | $R_f$ = 0.55 | $C_{25}H_{22}F_3NO_2$ (425) |

Beispiel 4

Allgemeine Vorschrift zur Herstellung der Verbindungen der allgemeinen Formel VII

Beispiel 4a

2Z-1-Hydroxy-2-fluor-3[4,6-bis-(4-fluorphenyl)-2-(1-methylethyl)-pyrimidin-3-yl]-propen-2

12,1 g (28,6 mMol) 2Z-2-Fluor-3[4,6-bis-(4-fluorphenyl)-2-(1-methylethyl)-primidin-3-yl]-acryl-säureäthylester (Bsp. 2a) werden in 200 ml abs. $CH_2Cl_2$ gelöst und bei 0 °C werden unter Argon 52,4 ml (62,4 mMol) Diisobutylaluminiumhydrid zugetropft. Es wird bei Raumtemperatur 2 Std. gerührt. Nach beendeter Reaktion wird das überschüssige Reagenz mit 10 ml Isopropanol zersetzt, dann gibt man 30 ml Wasser zu, rührt ca. 1/2 Stunde und filtriert über eine Klärschicht von ausgefallenen Aluminiumsalzen ab. Das Filtrat wird mit

$MgSO_4$ getrocknet, filtriert und eingeengt. Säulenchromatographie an Kieselgel (Cyclohexan/Essigester = 4 : 1) ergibt die Titelverbindung.

Ausbeute: 9,8 g weiße Kristalle (90 % d. Th.) VIIa Fp. 112-114 °C
$R_f$ = 0,2 (Cyclohexan/Essigester = 2 : 1)

$^1$H-NMR: δ-Werte in ppm. 1,38 (d, 6H, $CH_3$), 1,5 (s, 1H, OH), 3,28 (h, 1H, CH), 4,2 (dd, 2H J = 12Hz, $CH_2OH$), 5,92 (d, 1H J = 36Hz, $CH\overset{Z}{=}CF$), 7,1-7,2 (m, 4H. aromat. Prot.), 8,55-8,65 (m, 2H, aromat. Prot.)

E-Isomer: 3,7 (dd, 2H, J = 20Hz, $\underline{CH_2}OH$), 6,35 (d, 1H J = 16Hz, $CH\overset{E}{=}CF$)
MS: m/e 384 $C_{22}H_{19}ON_2F_3$

Beispiel 4b-4d

Die Verbindungen VII b-VII d wurden in analoger Weise, wie in Bsp. 4a beschrieben dargestellt (vgl. Tab. 3)

Tabelle 3

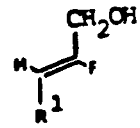 VII

| Beispiel | Verbindung | $R^1$ | Ausbeute % | $R_f$-Wert (Z) / Fp°C (Z) | $^1$H-NMR: δ/ppm = / MS : m/e = |
|---|---|---|---|---|---|
| 4 a | VIIa | 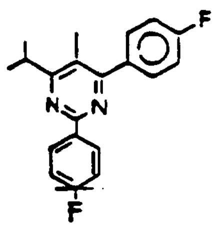 | 90 % | vgl. Beschreibung · Beispiel 4a | |
| 4 b | VIIb | 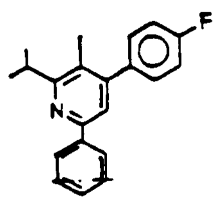 | 87 % | $R_f$ = 0.25 | $C_{23}H_{21}F_2NO$ (365) |
| 4 c | VII c | 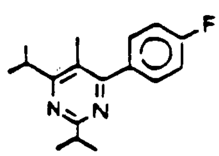 | 62 % | $R_f$ = 0.32 | $C_{19}H_{20}N_2F_2O$ (330) |
| 4 d | VII d | 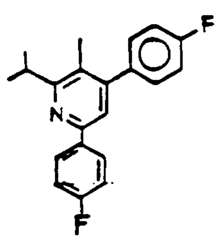 | 74 % | $R_f$ = 0.26 | $C_{23}H_{20}F_3NO$ (383) |

Beispiel 5

Allgemeine Vorschrift zur Herstellung von Verbindungen der allgemeinen Formel III

Beispiel 5a (Variante A)

2Z-2-Fluor-3[4,6-bis-(4-fluorphenyl)-2-(1-methylethyl)-pyrimidin-3-yl]-propen-2-aldehyd

11,3 g (97 mMol) N-Methyl-morpholin-N-oxid 97 %ig (Fluka) werden in 150 ml abs. Aceton gelöst. Bei 20 °C werden unter Rühren 0,9 g (0,97 mMol) Tris-(triphenylphosphin)-ruthenium-I-chlorid (Fluka) zugegeben. Anschließend werden 9,3 g (24,2 mMol) 2Z-1-Hydroxy-2-fluor-3[4,6-bis-(4-fluorphenyl)-2-(1-methylethyl)-pyrimidin-3-yl]-propen-2 (Bsp. 4a) in 150 ml abs. Aceton zugetropft. Es wird 20 Std. bei Raumtemperatur gerührt. Das Lösungsmittel wird im Vakuum entfernt und der Rückstand über Kieselgel filtriert (Diäthyläther mit 1 % $NEt_3$)

Ausbeute: 6,4 g (67,5 % d. Th.) weiße Kristalle IIIa Fp. 159-162 °C
$R_f$ = 0,89 (Cyclohexan/Essigester = 1 : 1)

$^1$H-NMR: δ-Werte in ppm: 1,38 (d, 6H, $CH_3$), 3,15 (h, 1H, CH), 6,9 (d, 1H, J = 34Hz, $CH\overset{Z}{=}CF$), 7,1-7,25 (m, 8H, aromat. Prot.), 7,6-7,7 (m, 2H, aromat. Prot.), 8,55-8,65 (m, 2H, aromat. Prot.), 9,4 (d, 1H J = 16Hz, CH=O).

MS: m/e 382 $C_{22}H_{17}N_2F_3O$

Beispiel 5b-5d

Die Verbindungen III b-III d wurden in analoger Weise, wie in Beispiel 5a beschrieben dargestellt (vgl. Tab. 4)

Die Verbindungen III a-III l können auch nach Variante B erhalten werden, wie im folgenden Beispiel 5a' beschrieben.

Beispiel 5a' (Variante B)

2Z-2-Fluor-3[4,6-bis-(4-fluorphenyl-2-(1-methylethyl)-pyrimidin-3-yl]-propen-2-aldehyd

7,2 g (20 mMol) 2-Fluor-3[4,6-bis-(4-fluorphenyl)-2-(1-methylethyl)-pyrimidin-3-yl]-acrylsäurenitril (Beispiel 3a) werden in 150 ml abs. THF gelöst. Bei -10 °C werden 35 ml (40 mMol) Diisobutylaluminiumhydrid (1,2 molare Lsg. in Toluol) zugetropft, in der Kälte wird 2 h weitergerührt. Dann wird mit 1 N Salzsäure auf pH 5-6 gestellt, mit Essigester extrahiert. Die vereinigten EE-Phasen werden mit $NaHCO_3$ säurefrei gewaschen, mit $MgSO_4$ getrocknet, filtriert und in Vak. eingeengt. Der Rückstand wird über Kieselgel chromatographiert. (Cyclohexan/Essigester = 10 : 1)

Ausbeute: Fraktion 16-60 3,1 g IIIa (E-Isomer)
$R_f$ = 0,45
Fraktion 61-120 3,1 g IIIa (Z-Isomer)
$R_f$ = 0,27
Fp. = 159-162 °C

$^1$H-NMR: Z-Isomer, identisch Bsp. 5a (Variante A)
δ = 6,92 ppm, J = 34Hz, CH=CF

$^1$H-NMR: E-Isomer
δ = 7,31 ppm, J = 16Hz, CH=CF

Tabelle 4

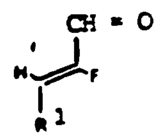

III

| Beispiel | Verbindung | $R^1$ | Ausbeute % | $R_f$-Wert (Z) Fp°C (Z) | $^1$H-NMR: δ/ppm = MS : m/e = |
|---|---|---|---|---|---|
| 5 a | IIIa | 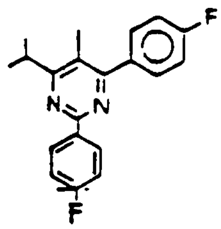 | 67.5 % | vgl. Beschreibung Beispiel 5a | |
| 5 b | IIIb | 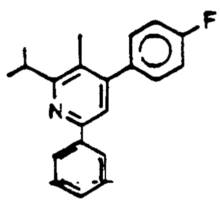 | 62.4 % | $R_f$ = 0.82 | $C_{23}H_{19}F_2NO$ (363) |
| 5 c | III c | 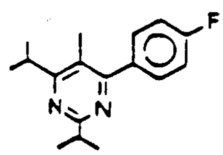 | 83 % | $R_f$ = 0.90 | $C_{19}H_{18}N_2F_2O$ (328) |
| 5 d | III d | 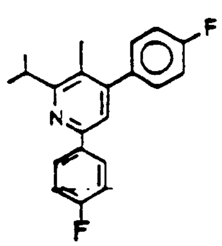 | 95 % | $R_f$ = 0.82 | $C_{23}H_{18}F_3NO$ (381) |

Beispiel 6

Allgemeine Vorschrift zur Herstellung von Verbindungen der allgemeinen Formel IV mit $R^{17}$ =

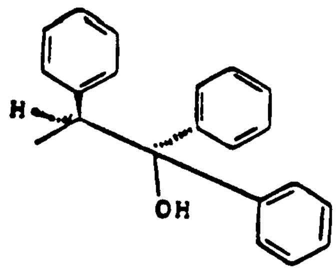

Beispiel 6a

4Z-3(S)Hydroxy-4-fluor-5-[4,6-bis-(4-fluorphenyl)-2-(1-methylethyl)-pyrimidin-3-yl]-pent-4-en-carbonsäure-2-(S)-Hytraester (Hytra = 2-Hydroxy-1,2,2-triphenylacetat)

Zu 1,5 g (2,1 ml, 13,5 mMol) Diisopropylamin abs. in 33 ml THF abs. werden bei 0 °C unter Rühren 9,3 ml Butyllithium (13,5 mMol) zugetropft. Dann rührt man noch 20 Min. bei 0 °C. Dann werden 2,21 g (6,6 mMol) (S)-(-)-2-Hydroxy-1, 2,2,-triphenylacetat (Merck-Schuchardt) in 50 ml abs. THF suspendiert bei -70 °C zu der Lithiumisopropylamidlösung getropft. Bei 0 °C wird weitergerührt bis eine klare organgerote Lösung entstanden ist. Bei -65 °C werden 2,4 g (6,4 mMol) 2Z-2-Fluor-3[4,6-bis-(4-fluorphenyl)-2-(1-methylethyl)-pyrimidin-3-yl]-propen-2-aldehyd (Bsp. 5a) in 20 ml als THF zugetropft und 1 Std. weitergerührt. Anschließend werden 20 ml halbgesättigte Ammoniumchloridlösung zugetropft, mit 2 x 50 ml Methylchlorid wird extrahiert und die org. Extrakte werden 2 x mit Wasser gewaschen, mit $MgSO_4$ getrocknet, filtriert und eingeengt.

Ausbeute: 5,2 g weiße Kristalle (87 % d. Th.)
Fp. 193-195 °C
$R_f$ = 0,29 (Cyclohexan/Essigester = 4 : 1)

$^1$H-NMR-$\delta$-Werte in ppm: 1,30 (dd, 6H, $CH_3$), 1,55 (s, 2H, OH), 2,45 und 2,82 (d, 2H, $CH_2$), 3,2 (h, 1H, CH), 4,3-4,45 (m, 1H, CHOH), 5,95 (d, 1H J = 38Hz, $CH\overset{Z}{=}CF$), 6,74 (s, 1H, CH-$C_6H_5$), 7,0-7,8 (m, 21H, aromat. Prot.), 8,5-8,65 (m, 2H, aromat. Prot.)

MS: m/e 714 $C_{44}H_{37}N_2F_3O_4$

Beispiel 6b-6d

Die Verbindungen VI b-VI d wurden in analoger Weise, wie in Beispiel 6a beschrieben dargestellt (vgl. Tab. 5)

Tabelle 5

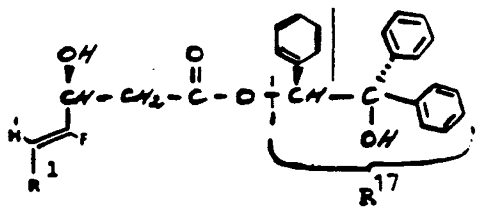

IV

| Beispiel | Verbindung | $R^1$ | Ausbeute % | $R_f$-Wert (Z) Fp°C (Z) | $^1H$-NMR: δ/ppm = MS: m/e = |
|---|---|---|---|---|---|
| 6 a | IVa | 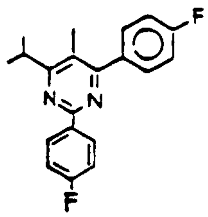 | 87 % | vgl. Beschreibung Beispiel 6a | |
| 6 b | IVb | 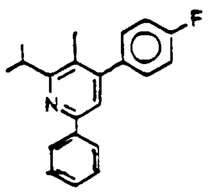 | 91 % | $R_f$ = 0.31 | $C_{45}H_{39}F_2NO_4$ (695) |
| 6 c | IVc | 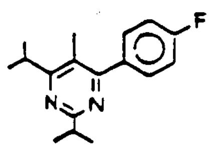 | 69 % | $R_f$ = 0.39 | $C_{41}H_{38}N_2F_2O_4$ (660) |
| 6 d | IV d | 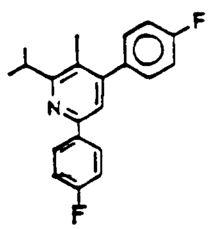 | 77 % | $R_f$ = 0.31 | $C_{45}H_{38}F_3NO_4$ (713) |

Beispiel 7

Allgemeine Vorschrift zur Herstellung von Verbindungen der allgemeinen Formel IV mit $R^{17} = CH_3$

Beispiel 7a

4Z-3(S)Hydroxy-4-fluor-5-[4,6-bis-(4-fluorphenyl)-2-(1-methylethyl)-pyrimidin-3-yl]-pent-4-en-carbonsäuremethylester

Zu einer Lösung von 184 mg (8 mMol) Natrium in 50 ml Methanol werden 5,2 g (7,3 mMol) 4Z-3(S)-Hydroxy-4-fluor-5-[4,6-bis-(4-fluorphenyl)-2-(1-methylethyl)-pyrimidin-3-yl]-pent-4-en-carbonsäure 2(S)-Hytraester (aus Bsp. 6a) in 50 ml abs. Methanol zugetropft. Es wird 16 Std. bei Raumtemperatur gerührt. Nach beendeter Reaktion werden 0,5 ml Eisessig zugegeben, mit 30 ml Toluol versetzt und i. Vak. eingeengt. Der Rückstand wird in Äther gelöst, 2 x mit Wasser gewaschen, die Ätherphase mit $MgSO_4$ getrocknet, filtriert und eingeengt. Filtration über Kieselgel mit Cyclohexan/Essigester = 4 : 1 ergibt

Ausbeute: 3,25 g (97,6 %) helles Öl
$R_f$ = 0,25 (Cyclohexan/Essigester 4 : 1)

$^1$H-NMR-$\delta$-Werte in ppm: 1,38 (d, $CH_3$, 6H), 2,05 (s, 1H, OH), 2,4-2,7 (m, 2H, $CH_2$), 3,25 (h, 1H, CH), 3,75 (s, 3H, $OCH_3$), 4,5-4,6 (m, 1H, CHOH), 6,1 (d, 1H J = 38Hz, $CH\overset{Z}{=}CF$), 7,05-7,2 (m, 4H, aromat. Prot.), 7,65-7,75 (m, 2H, aromat. Prot.), 8,55-8,65 (m, 2H, aromat. Prot.)

MS: m/e 456 $C_{25}H_{23}N_2F_3O_3$

Beispiel 7b-7d

Die Verbindungen IVe-IVh wurden in analoger Weise, wie in Beispiel 7a beschrieben, dargestellt (vgl. Tab. 6)

Tabelle 6

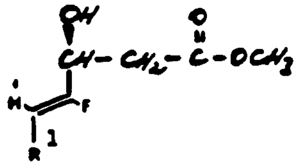 IV $R^{17}$ = $CH_3$

| Beispiel | Verbindung | $R^1$ | Ausbeute % | $R_f$-Wert (Z) Fp°C (Z) | $^1$H-NMR: δ/ppm = MS : m/e = |
|---|---|---|---|---|---|
| 7 a | IV e | 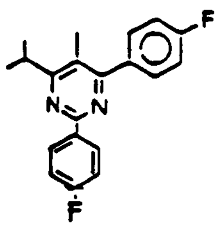 | 97.6 % | vgl. Beschreibung Beispiel 7a | |
| 7 b | IV f | 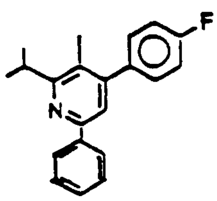 | 82 % | $R_f$ = 0.27 | $C_{26}H_{25}F_2NO_3$ (437) |
| 7 c | IV g | 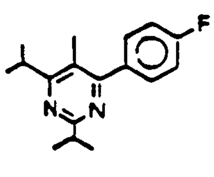 | 69 % | $R_f$ = 0.29 | $C_{22}H_{24}N_2FO_3$ (402) |
| 7d | IV h | 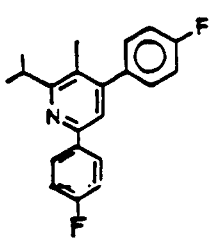 | 92 % | $R_f$ = 0.27 | $C_{26}H_{23}F_3O_3$ (455) |

Beispiel 8

Allgemeine Vorschrift zur Herstellung von Verbindungen der allgemeinen Formel V

Beispiel 8a

6Z-3-Oxo-5(S)Hydroxy-6-fluor-7[4,6-bis-[4-fluorphenyl)-2(1-methylethyl)-pyrimidin-3-yl]-hept-6-en-carbonsäure tert. butylester

Zu 2,02 g (2,8 ml, 20 mMol) Diisopropylamin in 12,5 ml abs. THF werden bei -70 °C 12,5 ml (29 mMol) Butyllithium (15 %ige Lösung in Hexan) getropft. Es wird 30 Min. bei 0 °C gerührt. Dann wird auf -70 °C abgekühlt und 2,33 g (20 mMol, 2,7 ml) Essigsäuretert.-butylester zugetropft und 30 Min. bei -70 °C weitergerührt. Dann werden 2,3 g (5 mMol) 4Z-3(S)Hydroxy-4-fluor-5-[4,6-bis-(4-fluorphenyl)-2(1-methylethyl)-pyrimidin-3-yl]-penten-4-en-carbonsäure methylester (aus Bsp. 7a) in 25 ml THF abs. bei -70 °C zugetropft. Anschließend läßt man auf R.T. erwärmen und versetzt mit 60 ml halb ges. Ammonchloridlösung. Mit 3 x 100 ml Methylenchlorid wird extrahiert. Die $CH_2Cl_2$-Extrakte werden mit Wasser gewaschen, mit $MgSO_4$ getrocknet, filtriert und i. Vak. eingeengt.

Ausbeute: 3,1 g helles Öl (82 % d.Th.)
$R_f$ = 0,54 (Cyclohexan/Essigester = 2 : 1)

$^1$H-NMR-δ-Werte in ppm: 1,38 (d, 6H, $CH_3$), 1,45 (s, 9H, $CH_3$), 1,6 (1H, s, OH), 2,5-2,9 (m, 2H, $CH_2$), 3,2-3,4 (m, 2H, $CH_2$), 4,6-4,7 (m, 1H, CHOH), 6,1 (s, 1H J = 38Hz, $CH\overset{Z}{=}CF$), 7,1-7,2 (m, 4H, aromat. Prot.), 7,65-7,75 (m, 2H, aromat. Prot.), 8,55-8,65 (m, 2H, aromat. Prot.)
MS: m/e = 540 $C_{30}H_{31}N_2F_3O_4$

Beispiel 8b-8d

Die Verbindungen Vb-Vd wurden in analoger Weise, wie in Beispiel 8a beschrieben, dargestellt (vgl. Tab. 7).

Tabelle 7

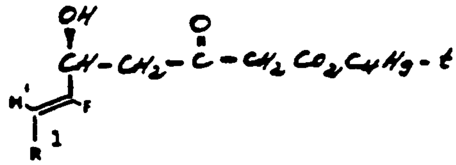 V

| Beispiel | Verbindung | $R^1$ | Ausbeute % | $R_f$-Wert (Z) / Fp°C (Z) | $^1$H-NMR: δ/ppm = / MS : m/e = |
|---|---|---|---|---|---|
| 8 a | Va | 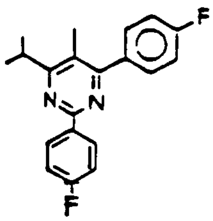 | 82 % | vgl. Beschreibung Beispiel 8a | |
| 8 b | Vb | 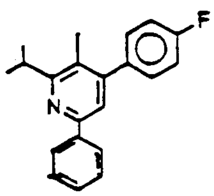 | 69 % | $R_f$ = 0.56 $C_{31}H_{43}F_2NO_4$ | (521) |
| 8 c | V c | 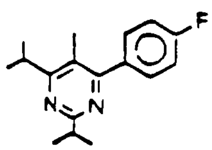 | 66 % | $R_f$ = 0.62 $C_{27}H_{32}N_2F_2O_4$ | (486) |
| 8 d | V d | 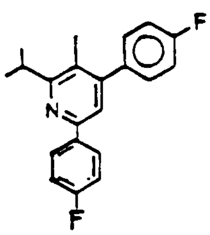 | 77 % | $R_f$ = 0.57 $C_{31}H_{32}F_3NO_4$ | (539) |

Beispiel 9

Allgemeine Vorschrift zur Herstellung von Verbindungen der allgemeinen Formel I ($R^2$ = t-$C_4H_9$)

Beispiel 9a

6Z-3(R), 5(S)Dihydroxy-6-fluor-7[4,6-bis(4-fluorphenyl)-2(1-methylethyl)-pyrimidin-3-yl]-hept-6-en-carbonsäure tert. butylester

Zu einer Lösung von 5,5 ml (5,5 mMol) Triäthylboran (1 Mol(ltr.) in abs. THF werden 10 ml Methanol in 40 ml abs. THF zugetropft und 1 Std. bei R.T. gerührt. Bei -70 °C werden 2,5 g (5 mMol) 6Z-3-Oxo-5(S)-hydroxy-6-fluor-7 [4,6-bis-(4-fluorphenyl)-2(1-methylethyl)-pyrimidin-3-yl]-hept-6-en carbonsäure tert. butylester (aus Bsp. 8a) in 20 ml abs. THF zugetropft und 30 Min. gerührt. Dann werden 210 mg (5,5 mMol) Natriumborhydrid zugegeben und bei -70 °C wird das Gemisch gerührt. Nach beendeter Reaktion wird in der Kälte mit 0,5 ml Eisessig in 3 ml Toluol versetzt und 5 Min. weitergerührt. Anschließend wird bei R.T. 50 ml halbgesättigte Natriumbicarbonatlösung zugesetzt und mit Methylenchlorid extrahiert. Die org. Extrakte werden mit $MgSO_4$ getrocknet, filtriert, im Vak. eingeengt und über Kieselgel filtriert (Cyclohexan/Essigester = 2 : 1)

Ausbeute: 3,0 g helle Kristalle (92,3 % d. Th.), Fp. 148 °C
$R_f$ = 0,325 (Cyclohexan/Essigester = 2 : 1)

$^1$H-NMR-δ-Werte in ppm: 1,35 (d, 6H, $CH_3$), 1,5 (s, 9H, $CH_3$), 1,42-1,6 (m, 2H, $CH_2$), 2,4 (d, 2H, $CH_2CO_2$-tert.but), 3,3 (h, 1H, CH), 3,9 (s, 1H, OH), 4,1 (s, 1H, OH), 4,12-4,28 (m, 1H, CHOH), 4,35-4,45 (m, 1H, CHOH), 6,08 (d, 1H, J = 38Hz, $CH\overset{Z}{=}CF$), 7,05-7,15 (m, 1H, aromat. Prot.), 7,7-7,8 (m, 1H, aromat. Prot.), 8,55-8,65 (m, 2H, aromat. Prot.).

MS: m/e = 540 $C_{30}H_{31}N_2F_3O_4$

Beispiel 9b-9d

Die Verbindungen I b-Id werden in analoger Weise, wie in Bsp. 9a beschrieben, dargestellt (vgl. Tab. 8).

Tabelle 8

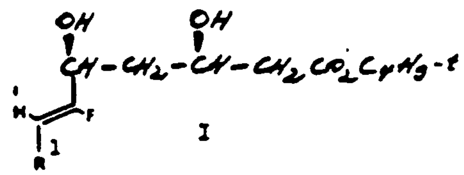

I

| Beispiel | Verbindung | $R^1$ | Ausbeute % | $R_f$-Wert (Z) / Fp°C (Z) | 1H-NMR: δ/ppm = / MS : m/e = |
|---|---|---|---|---|---|
| 9 a | Ia | 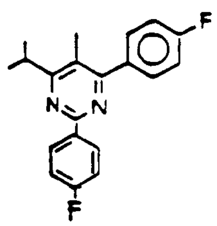 | 92.3 % | vgl. Beschreibung Beispiel 9a | |
| 9 b | Ib | 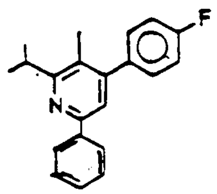 | 78 % | $R_f$ = 0.33 $C_{31}H_{45}F_2NO_4$ | (523) |
| 9 c | I c | 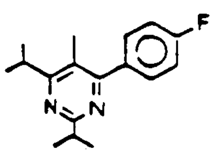 | 88 % | $R_f$ = 0.36 $C_{27}H_{34}N_2F_2O_4$ | (488) |
| 9 d | I d | 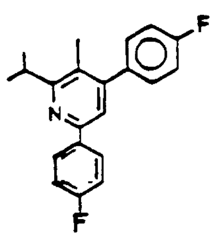 | 86 % | $R_f$ = 0.32 $C_{31}H_{34}F_3NO_4$ | (541) |

Beispiel 10

Allgemeine Vorschrift zur Herstellung von Verbindungen der allgemeinen Formel II

Beispiel 10a

Z-6(S)-2-[(4,6-bis-(4-fluorphenyl)-2-(1-methylethyl)-pyrimidin-3-yl)]-(1-fluor-2-ethenyl)-4(R)-hydroxy-3,4,5,6-tetrahydro-2H-pyran-2-on

2,7 g (5 mMol) 6Z-3(R), 5(S)-Dihydroxy-6-fluor-7[4,6-bis-(4-fluorphenyl)-2(1-methylethyl)-pyrimidin-3-yl]-hept-6-en cabronsäure tert. butylester (Bsp. 9a) werden in 50 ml Methylenchlorid gelöst und 3,99 g (2,7 ml, 35 mMol) Trifluoressigsäure zugegeben. Bei R.T. wird 6 Std. gerührt, danach mit Kaliumhydrogencarbonatlösung neutralisiert und mit Diäthylether 2 x extrahiert. Die Ätherextrakte werden mit $MgSO_4$ getrocknet, filtriert, eingeengt und über Kieselgel filtriert.

Ausbeute: 1,39 g (70 % d. Th.) weiße Kristalle Fp. 198 °C opt. Drehung: $[a]_D^{25}$ = -13,2 ° ($CH_3OH$, C = 1)
$R_f$ = 0,13 (cyclohexan/Essigester = 2 : 1)
$^1$H-NMR-δ-Werte in ppm: 1,38 (d, 6H, $CH_3$), 1,55 (s, 1H, OH), 1,8-2,0 (m, 2H, $CH_2$), 2,6-2,8 (m, 2H, $CH_2CO$), 3,25 (h, 1H, CH), 4,35-4,45 (m, 1H, CHOH), 5,15-5,35 (m, 1H, CHOCO), 6,1 (d, 1H J = 35Hz, $CH\overset{Z}{=}CF$), 7,1-7,2 (m, 4H, aromat. Prot.), 7,6-7,75 (m, 2H, aromat. Prot.), 8,5-8,6 (m, 2H, aromat. Prot.)
MS: m/e = 468 $C_{26}H_{23}N_2F_3O_3$

Beispiel 10b-10d

Die Verbindungen II b-II d wurden in analoger Weise, wie in Bsp. 10a beschrieben, dargestellt (vgl. Tab. 9).

Tabelle 9

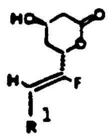 II

| Bei-spiel | Verbin-dung | $R^1$ | Ausbeute % | $R_f$-Wert (Z) Fp°C (Z) | $^1$H-NMR: δ/ppm = MS : m/e = |
|---|---|---|---|---|---|
| 10 a | IIa | 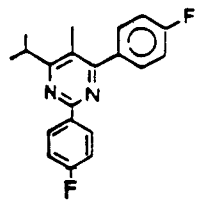 | 70 % | vgl. Beschreibung Beispiel 10a | |
| 10 b | IIb | 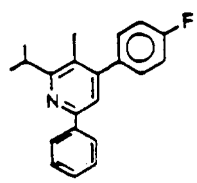 | 72 % | $R_f$ = 0.15 Fp. 165-167°C | $C_{27}H_{25}F_2NO_3$ (499) NRM: 1.35(d, 6H), 1.5-1.95(m, 2H), 2.55-2.80(m, 2H), 3.35(h,1H), 4.35-4.45(m,1H), 5.15-5.25(m,1H) 6.10 (d, 1H, J = 36Hz) 7.1-7.2(m,2H), 8.5-8.6(m,2H), 7.45-7.55 (m,3H) 7.65-7.75(m,2H) |
| 10 c | II c | 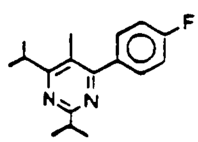 | 72 % | $R_f$ = 0.22 Fp. 138°C | $C_{23}H_{24}N_2F_2O_3$ (414) NMR: 1.25(d,6H), 1.38(d,6H), 1.75-2.0(m,2H), 1.5-1.7(breites s,1H), 2.6-2.8(m,2H), 3.1-3.3(m, 2H), 4.32-4.40(m,1H), 5.15-5.3(m,1H), 6.02 (d,J=36Hz,1H), 7.05-7.15(m,2H), 7.5-7.65(m,2H) |
| 10 d | II d | 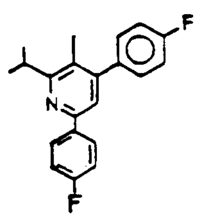 | 78 % | $R_f$ = 0.16 | $C_{27}H_{24}NF_3O_3$ (467) NMR: (analog Bsp. 10 b, 7.1-8.2(m, 8H) |

Beispiel 10 e

In analoger Weise wie in den vorhergehenden Beispielen beschrieben erhält man die Verbindung der Formel II worin $R^1$ einen Pyrimidinrest der Formel

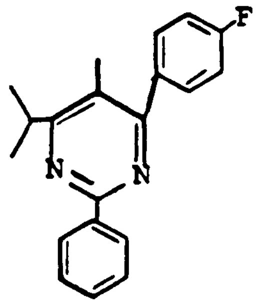

darstellt. Sie hat einen Schmelzpunkt von 195-197 °C.

Beispiel 11

Allgemeine Vorschrift zur Herstellung von Verbindungen der allgemeinen Formel I

$R^2$ = H

Beispiel 11a

6Z-3(R), 5(S)Dihydroxy-6-fluor-7[4,6-bis(4-fluorphenyl)-2 (1-methylethyl)-pyrimidin-3-yl]-hept-6-en-carbonsäure

Zu einer Lösung von 0,5 g 6Z-3(R), 5(S)Dihydroxy-6-fluor-7[4,6-bis(4-fluorphenyl)-2(1-methylethyl)-pyrimidin-3-yl]-hept-6-en-carbonsäure tert. butylester (Bsp. 9a) in 5 ml Äthanol gibt man 1 eq. 10 %ige wäßrige Natronlauge und rührt bei Raumtemperatur 3 Std. Dann wird in der Kälte mit 0,5 N Salzsäure auf pH 3-4 eingestellt, mit gesättigter Kochsalzlösung versetzt und 3 x mit Essigester extrahiert. Die org. Extrakte werden getrocknet mit $MgSO_4$, filtriert und im Vak. eingeengt.

Ausbeute: 0,42 g (92 % d. Th.) helles Öl
$R_f$ 0,13 (Cyclohexan/Essigester = 2 : 1)

$^1$H-NMR-$\delta$-Werte in ppm: (in DMSO) 1,3 (d, 6H, $CH_3$), 1,5-1,65 (m, 2H, $CH_2$), 1,75 (s, 1H, OH), 2,0-2,3 (m, 2H, $CH_2$), 3,3 (h, 1H, CH), 3,7-3,9 (m, 1H, CHOH), 4,15-4,3 (m, 1H, CHOH), 6,15 (d, 1H J = 36Hz, $CH\overset{Z}{=}CF$), 7,25-7,4 (m, 4H, aromat. Prot.), 7,8-7,9 (m, 2H, aromat. Prot.), 8,45-8,55 (m, 2H, aromat. Prot.), 5,5-5,9 (breites s, 1H, COOH)

MS: m/e =

Beispiel 11 b-11 d

Die Verbindungen der Formel I mit $R^2$ = H entsprechend Beispiel 11 b-11 d werden in analoger Weise wie in Bsp. 11a beschrieben dargestellt.

Unter den gleichen Reaktionsbedingungen wie in Beispiel 11a beschrieben lassen sich auch die Laktone der allgemeinen Formel II (Bsp. 10a) zu den freien Carbonsäuren bzw. deren Salzen verseifen.

## Patentansprüche

**Patentansprüche für folgende Vertragsstaaten : AT, BE, CH, DE, FR, GB, IT, LI, LU, NL, SE**

**1.** 6-Fluor-3,5-dihydroxycarbonsäuren und deren Derivate der allgemeinen Formel I

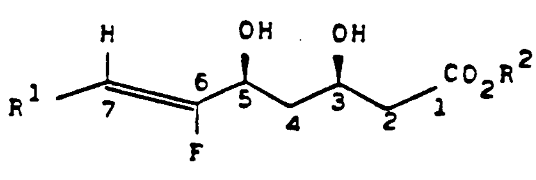

I

und die entsprechenden Laktone der Formel II

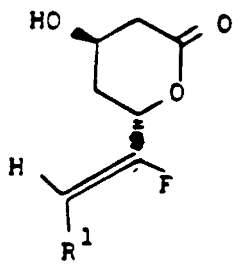

II

wobei in den allgemeinen Formeln I und II bedeuten

$R^1$ die Gruppe der substituierten 6-Ring-Heteroaromaten b

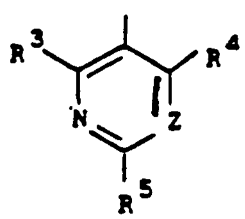

b

worin

Z einen Rest der Formel -CH oder ein Stickstoffatom

$R^3$, $R^4$, $R^5$ unabhängig voneinander Wasserstoff, einen geradkettigen oder verzweigten Alkyl- oder Alkenylrest mit jeweils bis zu 6 C-Atomen, der gegebenenfalls am terminalen Kohlenstoff durch einen Cycloalkyl- oder Cycloalkenylrest mit jeweils 3-6 C-Atomen substituiert sein kann, einen cyclischen gesättigten oder bis zu zweifach ungesättigten Kohlenwasserstoffrest mit 3-7 C-Atomen oder einen aromatischen Rest ausgewählt aus der Gruppe Phenyl, Furyl, Thienyl, Pyridinyl, der gegebenenfalls im Kern 1-3 gleiche oder verschiedene Substituenten der folgenden Gruppe tragen kann: Halogen,' Trifluormethyl, Alkyl oder Alkenyl mit je bis zu 6 C-Atomen, Hydroxy, Alkoxy mit 1-6 C-Atomen, Carboxy, Carbalkoxy mit 1-6 C-Atomen im Alkoxyteil, bedeuten, und

$R^2$ Wasserstoff, einen geradkettigen oder verzweigten Alkyl- oder Alkenylrest mit bis zu 8 C-Atomen, einen Benzylrest, dessen Kern 1-2 fach mit Halogen oder einem Alkylrest mit 1-4 C-Atomen substituiert sein kann, Alkalimetall oder ein Ammoniumion $NR^{13}R^{14}R^{15}R^{16}$, wobei $R^{13}$, $R^{14}$, $R^{15}$ und $R^{16}$ gleich oder verschieden sind und Wasserstoff, Alkyl mit 1-4 C-Atomen oder Hydroxyalkyl mit 1-4 C-Atomen bedeutet.

**2.** Verfahren zur Herstellung der Verbindungen der allgemeinen Formeln I und II, dadurch gekennzeichnet, daß man

a) entsprechend substituierte Aldehyde der Formel III

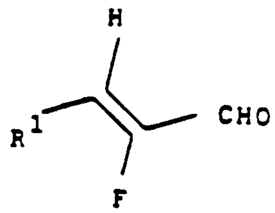

III

worin $R^1$ die in Anspruch 1 angegebene Bedeutung hat, in die entsprechenden Hydroxyester der allgemeinen Formel IV überführt,

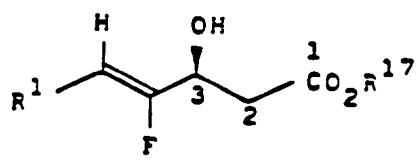

IV

worin $R^1$ die in Anspruch 1 angegebene Bedeutung hat und $R^{17}$ eine geeignete optisch aktive Säureschutzgruppe, die die Stereochemie an 3-C bestimmt, darstellt oder einen Alkylrest mit 1-8 C-Atomen

b) die optisch aktiven Verbindungen der Formel IV mit achiralen Essigsäureesterenolaten entweder direkt oder nach vorheriger Überführung in die entsprechenden Alkylester in die optisch aktiven Verbindungen der Formel V überführt,

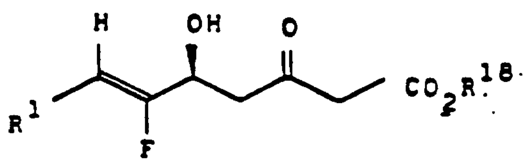

V

worin $R^1$ die in Anspruch 1 angegebene Bedeutung hat und $R^{18}$ Alkyl mit 1-8 C-Atomen ist,

c) die Hydroxyketoester der Formel V in die entsprechenden 6-Fluor-3,5-dihydroxyverbindungen der Formel I

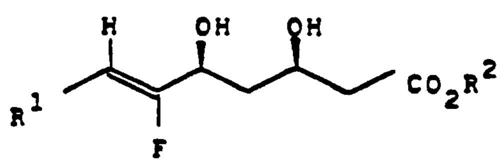

I

worin $R^1$ die zu Formel I in Anspruch 1 angegebene Bedeutung hat, und $R^2$ Alkyl mit 1-8 C-Atomen ist, überführt und eine erhaltene Verbindung gegebenenfalls verseift zu einer Verbindung der Formel I, worin $R^2$ ein Metallkation darstellt, daraus gegebenenfalls die freie Säure ($R^2$ = Wasserstoff) in Freiheit setzt und die freie Säure gegebenenfalls in Verbindungen der Formel I mit $R^2$ = Alkyl- oder Alkenyl mit bis zu 8 C-Atomen, Ammoniumion, Benzyl oder entsprechend substituiertes Benzyl überführt,

d) und eine erhaltene Verbindung der Formel I gegebenenfalls in ein Lakton der Formel II überführt

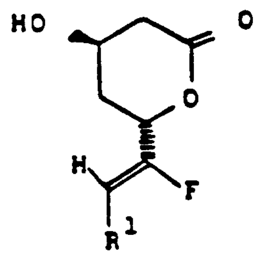

II

worin $R^1$ die in Anspruch 1 angegebene Bedeutung hat.

3. Pharmazeutisches Präparat, dadurch gekennzeichnet, daß es eine Verbindung gemäß Anspruch 1 enthält.

4. Verbindungen gemäß Anspruch 1 zur Prophylaxe und Therapie der Arteriosklerose und Hypercholesterinamie.

5. Verbindungen der Formel III

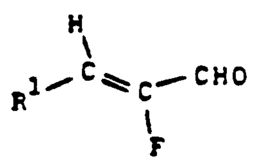

III

worin $R^1$ die zur Formel I in Anspruch 1 angegebene Bedeutung hat.

6. Verbindungen der Formel IV

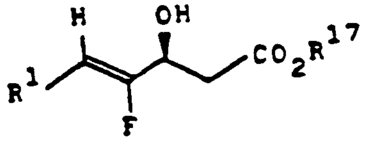

IV

worin $R^1$ die zur Formel I in Anspruch 1 angegebene Bedeutung hat und $R_{17}$ Alkyl mit 1-8 C-Atomen ist.

7. Verbindungen der Formel V

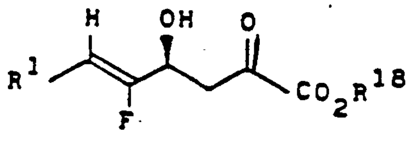

V

worin $R^1$ die zur Formel I in Anspruch 1 angegebene Bedeutung hat und $R^{18}$ Alkyl mit 1-8 C-Atomen ist.

**8.** Verbindungen der Formel VI

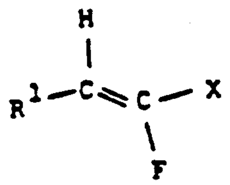

VI

worin $R^1$ die zur Formel I in Anspruch 1 angegebene Bedeutung hat und X eine Nitrilgruppe (C≡N) oder eine Estergruppe $COOR^{19}$ ist, wobei $R^{19}$ Alkyl mit 1-8 C-Atomen ist.

**9.** Verbindungen der Formel VII

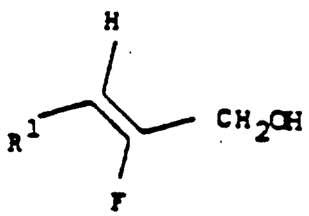

VII

worin $R^1$ die zu Formel I in Anspruch 1 angegebene Bedeutung hat.

**Patentansprüche für folgenden Vertragsstaat : ES**

**1.** Verfahren zur Herstellung von 6-Fluor-3,5-dihydroxycarbonsäuren und deren Derivaten der allgemeinen Formel I

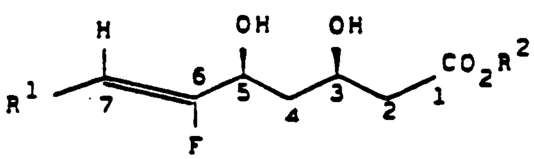

I

und von den entsprechenden Laktonen der Formel II

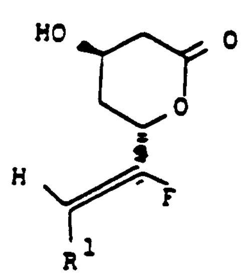

wobei in den allgemeinen Formeln I und II bedeuten

$R^1$ die Gruppe der substituierten 6-Ring-Heteroaromaten b.

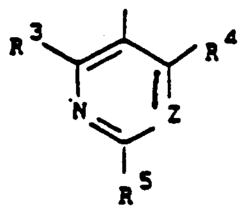

b

worin

Z einen Rest der Formel -CH oder ein Stickstoffatom

$R^3$, $R^4$, $R^5$ unabhängig voneinander Wasserstoff, einen geradkettigen oder verzweigten Alkyl- oder Alkenylrest mit jeweils bis zu 6 C-Atomen, der gegebenenfalls am terminalen Kohlenstoff durch einen Cycloalkyl- oder Cycloalkenylrest mit jeweils 3-6 C-Atomen substituiert sein kann, einen cyclischen gesättigten oder bis zu zweifach ungesättigten Kohlenwasserstoffrest mit 3-7 C-Atomen oder einen aromatischen Rest ausgewählt aus der Gruppe Phenyl, Furyl, Thienyl, Pyridinyl, der gegebenenfalls im Kern 1-3 gleiche oder verschiedene Substituenten der folgenden Gruppe tragen kann: Halogen, Trifluormethyl, Alkyl oder Alkenyl mit je bis zu 6 C-Atomen, Hydroxy, Alkoxy mit 1-6 Atomen, Carboxy, Carbalkoxy mit 1-6 C-Atomen im Alkoxyteil, bedeuten, und

$R^2$ Wasserstoff, einen geradkettigen oder verzweigten Alkyl- oder Alkenylrest mit bis zu 8 C-Atomen, einen Benzylrest, dessen Kern 1-2 fach mit Halogen oder einem Alkylrest mit 1-4 C-Atomen substituiert sein kann, Alkalimetall oder ein Ammoniumion $NR^{13}R^{14}R^{15}R^{16}$, wobei $R^{13}$, $R^{14}$, $R^{15}$ und $R^{16}$ gleich oder verschieden sind und Wasserstoff, Alkyl mit 1-4 C-Atomen oder Hydroxyalkyl mit 1-4 C-Atomen bedeutet,

dadurch gekennzeichnet, daß man

a) entsprechend substituierte Aldehyde der Formel III

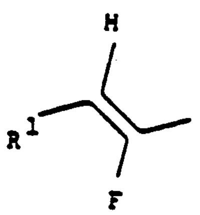

III

worin $R^1$ die angegebene Bedeutung hat, in die entsprechenden Hydroxyester der allgemeinen Formel IV überführt,

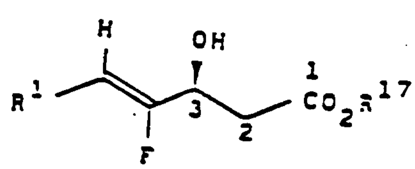

IV

worin $R^1$ die angegebene Bedeutung hat und $R^{17}$ eine geeignete optisch aktive Säureschutzgruppe, die die Stereochemie an 3-C bestimmt, darstellt oder einen Alkylrest mit 1-8 C-Atomen

b) die optisch aktiven Verbindungen der Formel IV mit achiralen Essigsäureesterenolaten entweder direkt oder nach vorheriger Überführung in die entsprechenden Alkylester in die optisch aktiven Verbindungen der Formel V überführt,

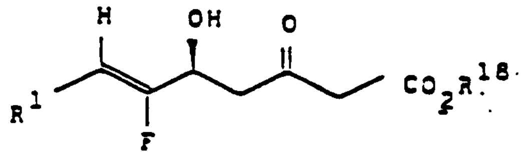

V

worin $R^1$ die angegebene Bedeutung hat und $R^{18}$ Alkyl mit 1-8 C-Atomen ist,
c) die Hydroxyketoester der Formel V in die entsprechenden 6-Fluor-3,5-dihydroxyverbindungen der Formel I

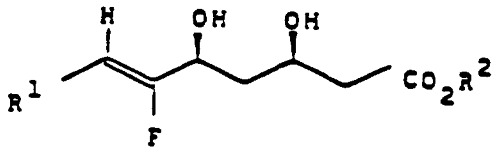

I

worin $R^1$ die zu Formel I angegebene Bedeutung hat, und $R^2$ Alkyl mit 1-8 C-Atomen ist, überführt und eine erhaltene Verbindung gegebenenfalls verseift zu einer Verbindung der Formel I, worin $R^2$ ein Metallkation darstellt, daraus gegebenenfalls die freie Säure ($R^2$ = Wasserstoff) in Freiheit setzt und die freie Säure gegebenenfalls in Verbindungen der Formel I mit $R^2$ = Alkyl- oder Alkenyl mit bis zu 8 C-Atomen, Ammoniumion, Benzyl oder entsprechend substituiertes Benzyl überführt,
d) und eine erhaltene Verbindung der Formel I gegebenenfalls in ein Lakton der Formel II überführt

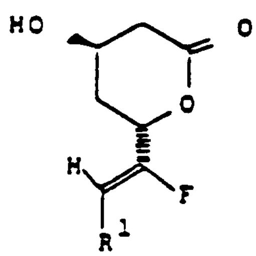

II

worin $R^1$ die angegebene Bedeutung hat.

**2.** Verfahren zur Herstellung eines pharmazeutischen Präparates, dadurch gekennzeichnet, daß man eine Verbindung erhältlich nach Anspruch 1 in eine geeignete Darreichungsform überführt.

**3.** Verfahren gemäß Anspruch 1, dadurch gekennzeichnet, daß man eine Verbindung der Formel III

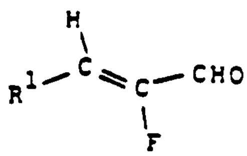

III

worin $R^1$ die zur Formel I in Anspruch 1 angegebene Bedeutung hat, isoliert.

4. Verfahren gemäß Anspruch 1, dadurch gekennzeichnet, daß man eine Verbindung der Formel IV

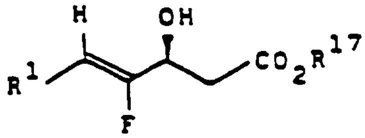

IV

worin $R^1$ die zur Formel I in Anspruch 1 angegebene Bedeutung hat und $R_{17}$ Alkyl mit 1-8 C-Atomen ist, isoliert.

5. Verfahren gemäß Anspruch 1, dadurch gekennzeichnet, daß man eine Verbindung der Formel V

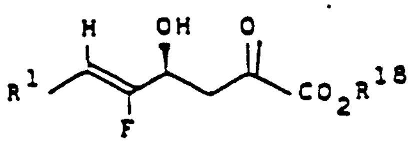

V

worin $R^1$ die zur Formel I in Anspruch 1 angegebene Bedeutung hat und $R^{18}$ Alkyl mit 1-8 C-Atomen ist, isoliert.

6. Verfahren gemäß Anspruch 1, dadurch gekennzeichnet, daß man eine Verbindung der Formel VI

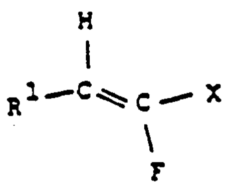

VI

worin $R^1$ die zur Formel I in Anspruch 1 angegebene Bedeutung hat und X eine Nitrilgruppe (C≡N) oder eine Estergruppe $COOR^{19}$ ist, wobei $R^{19}$ Alkyl mit 1-8 C-Atomen ist, isoliert.

7. Verfahren gemäß Anspruch 1, dadurch gekennzeichnet, daß man eine Verbindung der Formel VII

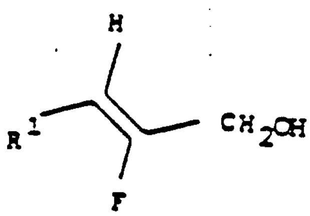

VII

worin $R^1$ die zu Formel I in Anspruch 1 angegebene Bedeutung hat, isoliert.

**Patentansprüche für folgenden Vertragsstaat : GR**

1. Verfahren zur Herstellung von 6-Fluor-3,5-dihydroxycarbonsäuren und deren Derivaten der allgemeinen Formel I

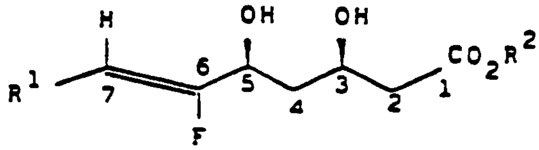

I

und von den entsprechenden Laktonen der Formel II

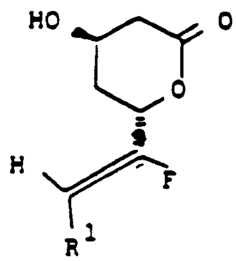

II

worin in den allgemeinen Formeln I und II bedeuten

$R^1$ die Gruppe der substituierten 6-Ring-Heteroaromaten b

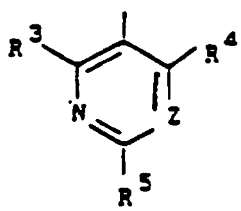

b

worin

Z einen Rest der Formel -CH oder ein Stickstoffatom

$R^3$, $R^4$, $R^5$ unabhängig voneinander Wasserstoff, einen geradkettigen oder verzweigten Alkyl- oder Alkenylrest mit jeweils bis zu 6 C-Atomen, der gegebenenfalls am terminalen Kohlenstoff durch einen Cycloalkyl- oder Cycloalkenylrest mit jeweils 3-6 C-Atomen substituiert sein kann, einen cyclischen gesättigten oder bis zu zweifach ungesättigten Kohlenwasserstoffrest mit 3-7 C-Atomen oder einen aromatischen Rest ausgewählt aus der Gruppe Phenyl, Furyl, Thienyl, Pyridinyl, der gegebenenfalls im Kern 1-3 gleiche oder verschiedene Substituenten der folgenden Gruppe tragen kann: Halogen, Trifluormethyl, Alkyl oder Alkenyl mit je bis zu 6 C-Atomen, Hydroxy, Alkoxy mit 1-6 Atomen, Carboxy, Carbalkoxy mit 1-6 C-Atomen im Alkoxyteil, bedeuten, und

$R^2$ Wasserstoff, einen geradkettigen oder verzweigten Alkyl- oder Alkenylrest mit bis zu 8 C-Atomen, einen Benzylrest, dessen Kern 1-2 fach mit Halogen oder einem Alkylrest mit 1-4 C-Atomen substituiert sein kann, Alkalimetall oder ein Ammoniumion $NR^{13}R^{14}R^{15}R^{16}$, wobei $R^{13}$, $R^{14}$, $R^{15}$ und $R^{16}$ gleich oder verschieden sind und Wasserstoff, Alkyl mit 1-4 C-Atomen oder Hydroxyalkyl mit 1-4 C-Atomen bedeutet,

dadurch gekennzeichnet, daß man

a) entsprechend substituierte Aldehyde der Formel III

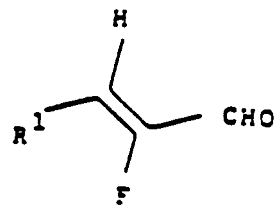

III

worin $R^1$ die angegebene Bedeutung hat, in die entsprechenden Hydroxyester der allgemeinen Formel IV überführt,

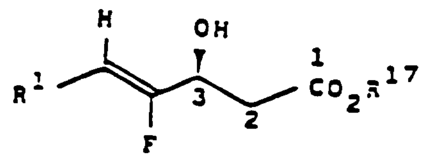

IV

worin $R^1$ die angegebene Bedeutung hat und $R^{17}$ eine geeignete optisch aktive Säureschutzgruppe, die die Stereochemie an 3-C bestimmt, darstellt oder einen Alkylrest mit 1-8 C-Atomen

b) die optisch aktiven Verbindungen der Formel IV mit achiralen Essigsäureesterenolaten entweder direkt oder nach vorheriger Überführung in die entsprechenden Alkylester in die optisch aktiven Verbindungen der Formel V überführt,

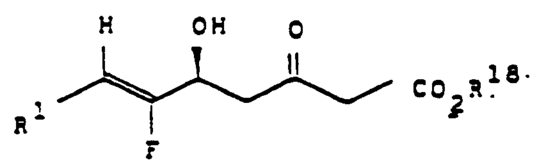

V

worin $R^1$ die angegebene Bedeutung hat und $R^{18}$ Alkyl mit 1-8 C-Atomen ist,

c) die Hydroxyketoester der Formel V in die entsprechenden 6-Fluor-3,5-dihydroxyverbindungen der Formel I

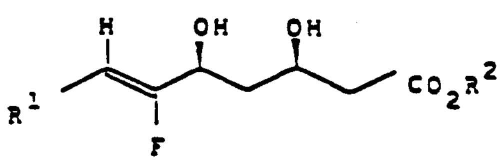

I

worin $R^1$ die zu Formel I angegebene Bedeutung hat, und $R^2$ Alkyl mit 1-8 C-Atomen ist, überführt und eine erhaltene Verbindung gegebenenfalls verseift zu einer Verbindung der Formel I, worin $R^2$ ein Metallkation darstellt, daraus gegebenenfalls die freie Säure ($R^2$ = Wasserstoff) in Freiheit setzt und die freie Säure gegebenenfalls in Verbindungen der Formel I mit $R^2$ = Alkyl- oder Alkenyl mit bis zu 8 C-Atomen, Ammoniumion, Benzyl oder entsprechend substituiertes Benzyl überführt,

d) und eine erhaltene Verbindung der Formel I gegebenenfalls in ein Lakton der Formel II überführt

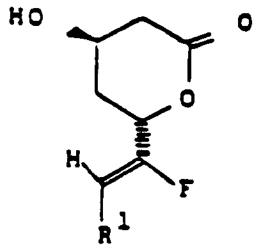

II

worin $R^1$ die angegebene Bedeutung hat.

2. Verfahren zur Herstellung eines pharmazeutischen Präparates, dadurch gekennzeichnet, daß man eine Verbindung erhältlich nach Anspruch 1 in eine geeignete Darreichungsform überführt.

3. Verbindungen der Formel III

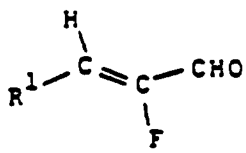

III

worin $R^1$ die zur Formel I in Anspruch 1 angegebene Bedeutung hat.

4. Verbindungen der Formel IV

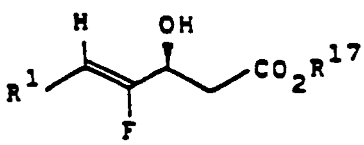

IV

worin $R^1$ die zur Formel I in Anspruch 1 angegebene Bedeutung hat und $R_{17}$ Alkyl mit 1-8 C-Atomen ist.

5. Verbindungen der Formel V

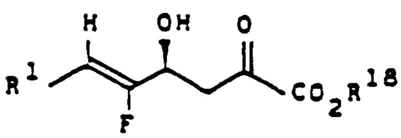

V

worin $R^1$ die zur Formel I in Anspruch 1 angegebene Bedeutung hat und $R^{18}$ Alkyl mit 1-8 C-Atomen ist.

**6.** Verbindungen der Formel VI

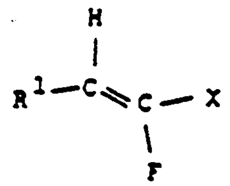

VI

worin $R^1$ die zur Formel I in Anspruch 1 angegebene Bedeutung hat und X eine Nitrilgruppe (C≡N) oder eine Estergruppe $COOR^{19}$ ist, wobei $R^{19}$ Alkyl mit 1-8 C-Atomen ist.

**7.** Verbindungen der Formel VII

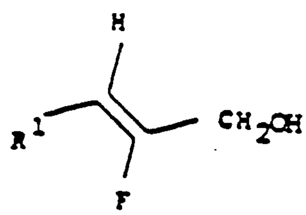

VII

worin $R^1$ die zu Formel I in Anspruch 1 angegebene Bedeutung hat.

**Claims**

**Claims for the following Contracting States : AT, BE, CH, DE, FR, GB, IT, LI, LU, NL, SE**

**1.** 6-Fluoro-3,5-dihydroxy carboxylic acids and the derivatives thereof of the formula I

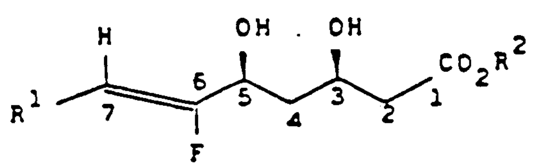

I

and the corresponding lactones of the formula II

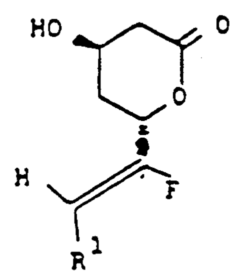

where, in the formulae I and II,

$R^1$ denotes the group of substituted 6-membered ring heteroaromatics b

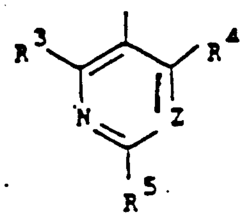

b

in which

| | |
|---|---|
| Z | denotes a radical of the formula -CH or a nitrogen atom, |
| $R^3$, $R^4$ and $R^5$ | independently of one another denote hydrogen, a straight-chain or branched alkyl or alkenyl radical, each of which has up to 6 carbon atoms and which can optionally be substituted on the terminal carbon by a cycloalkyl or cycloalkenyl radical, each of which has 3-6 carbon atoms, or denote a cyclic hydrocarbon radical which is saturated or up to doubly unsaturated and has 3-7 carbon atoms, or an aromatic radical selected from the group comprising phenyl, furyl, thienyl and pyridinyl, which can optionally carry in the nucleus 1-3 identical or different substituents from the following group: halogen, trifluoromethyl, alkyl or alkenyl, each of which has up to 6 carbon atoms, hydroxyl, alkoxy having 1-6 carbon atoms, carboxyl or carbalkoxy having 1-6 carbon atoms in the alkoxy moiety, and |
| $R^2$ | denotes hydrogen, a straight-chain or branched alkyl or alkenyl radical, each of which has up to 8 carbon atoms, a benzyl radical whose nucleus can be substituted 1-2 times by halogen or an alkyl radical having 1-4 carbon atoms, or denotes alkali metal or an ammonium ion $NR^{13}R^{14}R^{15}R^{16}$, where $R^{13}$, $R^{14}$, $R^{15}$ and $R^{16}$ are identical or different and denote hydrogen, alkyl having 1-4 carbon atoms or hydroxyalkyl having 1-4 carbon atoms. |

**2.** A process for the preparation of the compounds of the formulae I and II, which comprises
a) converting appropriately substituted aldehydes of the formula III

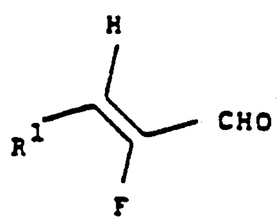

III

in which $R^1$ has the specified meaning, into the corresponding hydroxy esters of the formula IV

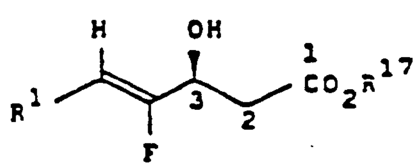

IV

in which $R^1$ has the specified meaning, and $R^{17}$ represents a suitable optically active acid protecting group which determines the stereochemistry at C-3, or an alkyl radical having 1-8 carbon atoms,
b) converting the optically active compounds of the formula IV with achiral acetic ester enolates, either directly or after previous conversion into the corresponding alkyl esters, into the optically active compounds of the formula V

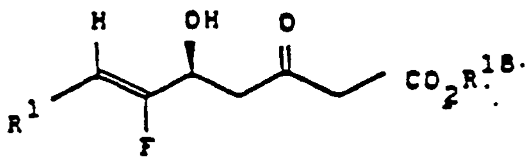

V

in which $R^1$ has the specified meaning, and $R^{18}$ is alkyl having 1-8 carbon atoms,

c) converting the hydroxy keto esters of the formula V into the corresponding 6-fluoro-3,5-dihydroxy compounds of the formula I

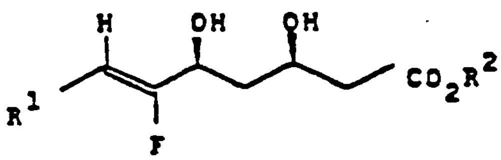

I

in which $R^1$ has the meaning specified for formula I and $R^2$ is alkyl having 1-8 carbon atoms, and, where appropriate, hydrolyzing a resulting compound to a compound of the formula I in which $R^2$ represents a metal cation, where appropriate liberating therefrom the free acid ($R^2$ = hydrogen), and where appropriate converting the free acid into compounds of the formula I with $R^2$ = alkyl or alkenyl, each having up to 8 carbon atoms, ammonium ion, benzyl or appropriately substituted benzyl,

d) and converting a resulting compound of the formula I where appropriate into a lactone of the formula II

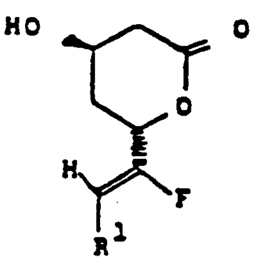

II

in which $R^1$ has the meaning specified in claim 1.

**3.** A pharmaceutical product which contains a compound as claimed in claim 1.

**4.** A compound as claimed in claim 1 for the prophylaxis and therapy of arteriosclerosis and hypercholesterolemia.

**5.** A compound of the formula III

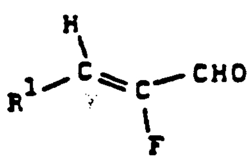

III

in which $R^1$ has the meaning specified for formula I in claim 1.

6. A compound of the formula IV

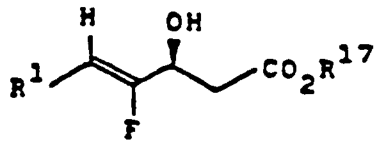

IV

in which $R^1$ has the meaning specified for formula I in claim 1, and $R^{17}$ is alkyl having 1-8 carbon atoms.

7. A compound of the formula V

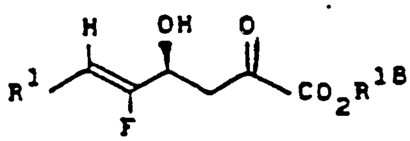

V

in which $R^1$ has the meaning specified for formula I in claim 1, and $R^{18}$ is alkyl having 1-8 carbon atoms.

8. A compound of the formula VI

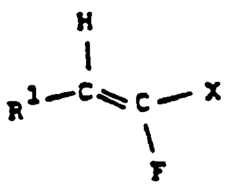

VI

in which $R^1$ has the meaning specified for formula I in claim 1, and X is a nitrile group (C≡N) or an ester group $COOR^{19}$, where $R^{19}$ is alkyl having 1-8 carbon atoms.

9. A compound of the formula VII

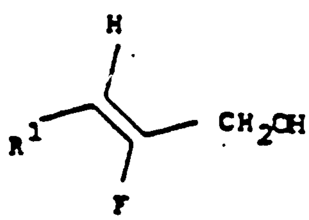

VII

in which $R^1$ has the meaning specified for formula I in claim 1.

**Claims for the following Contracting State : ES**

1. A process for the preparation of 6-fluoro-3,5-dihydroxy carboxylic acids and the derivatives thereof of the formula I

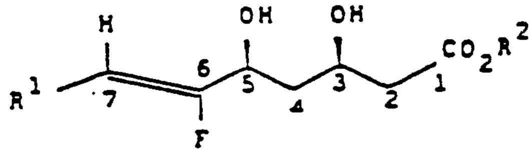

I

and of the corresponding lactones of the formula II

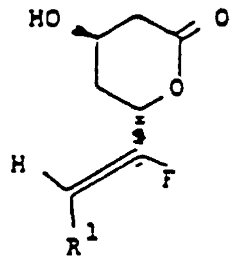

II

where, in the formulae I and II,

$R^1$ denotes the group of substituted 6-membered ring heteroaromatics b

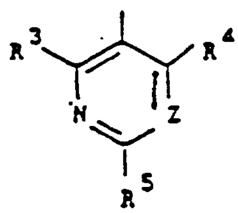

b

in which

Z denotes a radical of the formula -CH or a nitrogen atom,

$R^3$, $R^4$ and $R^5$ independently of one another denote hydrogen, a straight-chain or branched alkyl or alkenyl radical, each of which has up to 6 carbon atoms and which can optionally be substituted on the terminal carbon by a cycloalkyl or cycloalkenyl radical, each of which has 3-6 carbon atoms, or denote a cyclic hydrocarbon radical which is saturated or up to doubly unsaturated and has 3-7 carbon atoms, or an aromatic radical selected from the group comprising phenyl, furyl, thienyl and pyridinyl, which can optionally carry in the nucleus 1-3 identical or different substituents from the following group: halogen, trifluoromethyl, alkyl or alkenyl, each of which has up to 6 carbon atoms, hydroxyl, alkoxy having 1-6 carbon atoms, carboxyl or carbalkoxy having 1-6 carbon atoms in the alkoxy moiety, and

$R^2$ denotes hydrogen, a straight-chain or branched alkyl or alkenyl radical, each of which has up to 8 carbon atoms, a benzyl radical whose nucleus can be substituted 1-2 times by halogen or an alkyl radical having 1-4 carbon atoms, or denotes alkali metal or an ammonium ion $NR^{13}R^{14}R^{15}R^{16}$, where $R^{13}$, $R^{14}$, $R^{15}$ and $R^{16}$ are identical or different and denote hydrogen, alkyl having 1-4 carbon atoms or hydroxyalkyl having 1-4 carbon atoms, which comprises

a) converting appropriately substituted aldehydes of the formula III

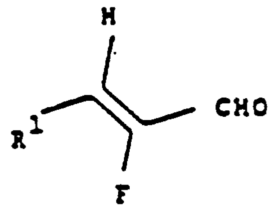

III

in which $R^1$ has the specified meaning, into the corresponding hydroxy esters of the formula IV

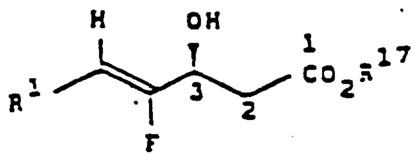

IV

in which $R^1$ has the specified meaning, and $R^{17}$ represents a suitable optically active acid protecting group which determines the stereochemistry at C-3, or an alkyl radical having 1-8 carbon atoms,

b) converting the optically active compounds of the formula IV with achiral acetic ester enolates, either directly or after previous conversion into the corresponding alkyl esters, into the optically active compounds of the formula V

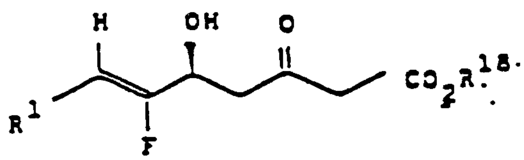

V

in which $R^1$ has the specified meaning, and $R^{18}$ is alkyl having 1-8 carbon atoms,

c) converting the hydroxy keto esters of the formula V into the corresponding 6-fluoro-3,5-dihydroxy compounds of the formula I

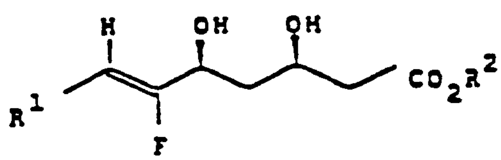

I

in which $R^1$ has the meaning specified for formula I and $R^2$ is alkyl having 1-8 carbon atoms, and, where appropriate, hydrolyzing a resulting compound to a compound of the formula I in which $R^2$ represents a metal cation, where appropriate liberating therefrom the free acid ($R^2$ = hydrogen), and where appropriate converting the free acid into compounds of the formula I with $R^2$ = alkyl or alkenyl, each having up to 8 carbon atoms, ammonium ion, benzyl or appropriately substituted benzyl,

d) and converting a resulting compound of the formula I where appropriate into a lactone of the formula II

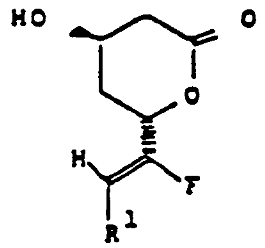

II

in which $R^1$ has the specified meaning.

**2.** A process for the preparation of a pharmaceutical product, which comprises converting a compound obtainable as claimed in claim 1 into a suitable administration form.

**3.** The process as claimed in claim 1, which comprises isolating a compound of the formula III

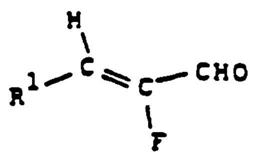

III

in which $R^1$ has the meaning specified for formula I in claim 1.

**4.** The process as claimed in claim 1, which comprises isolating a compound of the formula IV

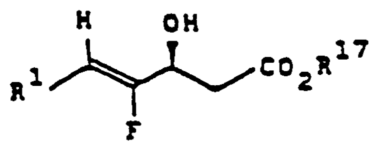

IV

in which $R^1$ has the meaning specified for formula I in claim 1, and $R^{17}$ is alkyl having 1-8 carbon atoms.

**5.** The process as claimed in claim 1, which comprises isolating a compound of the formula V

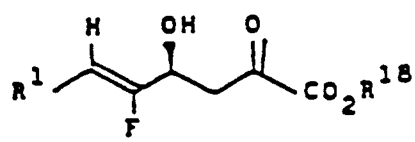

V

in which $R^1$ has the meaning specified for formula I in claim 1, and $R^{18}$ is alkyl having 1-8 carbon atoms.

6. The process as claimed in claim 1, which comprises isolating a compound of the formula VI

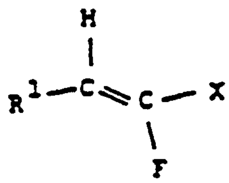

VI

in which $R^1$ has the meaning specified for formula I in claim 1, and X is a nitrile group (C≡N) or an ester group $COOR^{19}$, where $R^{19}$ is alkyl having 1-8 carbon atoms.

7. The process as claimed in claim 1, which comprises isolating a compound of the formula VII

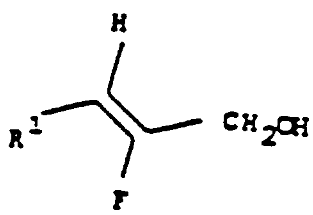

VII

in which $R^1$ has the meaning specified for formula I in claim 1.

**Claims for the following Contracting State : GR**

1. A process for the preparation of 6-fluoro-3,5-dihydroxy carboxylic acids and the derivatives thereof of the formula I

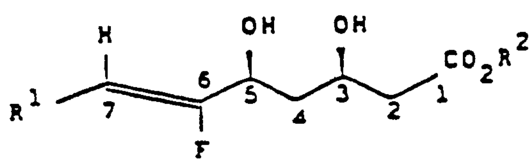

I

and of the corresponding lactones of the formula II

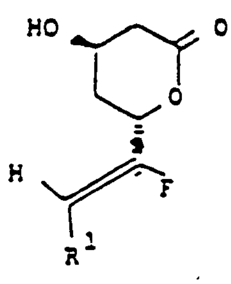

II

where, in the formulae I and II,

$R^1$ denotes the group of substituted 6-membered ring heteroaromatics b

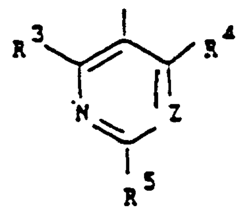

b

in which

Z denotes a radical of the formula -CH or a nitrogen atom,

$R^3$, $R^4$ and $R^5$ independently of one another denote hydrogen, a straight-chain or branched alkyl or alkenyl radical, each of which has up to 6 carbon atoms and which can optionally be substituted on the terminal carbon by a cycloalkyl or cycloalkenyl radical, each of which has 3-6 carbon atoms, or denote a cyclic hydrocarbon radical which is saturated or up to doubly unsaturated and has 3-7 carbon atoms, or an aromatic radical selected from the group comprising phenyl, furyl, thienyl and pyridinyl, which can optionally carry in the nucleus 1-3 identical or different substituents from the following group: halogen, trifluoromethyl, alkyl or alkenyl, each of which has up to 6 carbon atoms, hydroxyl, alkoxy having 1-6 carbon atoms, carboxyl or carbalkoxy having 1-6 carbon atoms in the alkoxy moiety, and

$R^2$ denotes hydrogen, a straight-chain or branched alkyl or alkenyl radical, each of which has up to 8 carbon atoms, a benzyl radical whose nucleus can be substituted 1-2 times by halogen or an alkyl radical having 1-4 carbon atoms, or denotes alkali metal or an ammonium ion $NR^{13}R^{14}R^{15}R^{16}$, where $R^{13}$, $R^{14}$, $R^{15}$ and $R^{16}$ are identical or different and denote hydrogen, alkyl having 1-4 carbon atoms or hydroxyalkyl having 1-4 carbon atoms, which comprises

a) converting appropriately substituted aldehydes of the formula III

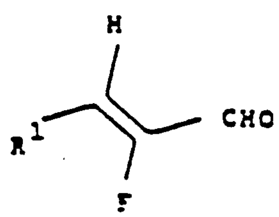

III

in which $R^1$ has the specified meaning, into the corresponding hydroxy esters of the formula IV

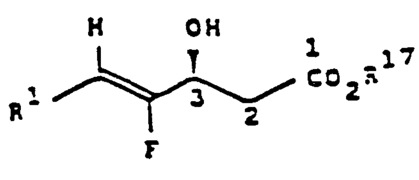

IV

in which $R^1$ has the specified meaning, and $R^{17}$ represents a suitable optically active acid protecting group which determines the stereochemistry at C-3, or an alkyl radical having 1-8 carbon atoms,

b) converting the optically active compounds of the formula IV with achiral acetic ester enolates, either directly or after previous conversion into the corresponding alkyl esters, into the optically active compounds of the formula V

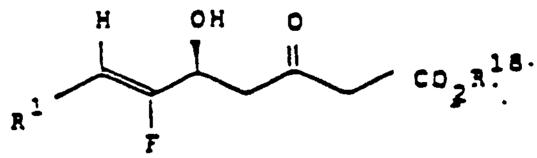

V

in which $R^1$ has the specified meaning, and $R^{18}$ is alkyl having 1-8 carbon atoms,
c) converting the hydroxy keto esters of the formula V into the corresponding 6-fluoro-3,5-dihydroxy compounds of the formula I

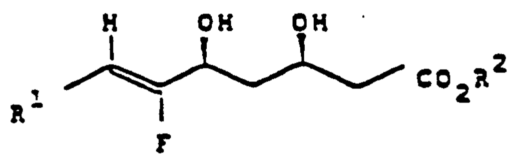

I

in which $R^1$ has the meaning specified for formula I , and $R^2$ is alkyl having 1-8 carbon atoms, and, where appropriate, hydrolyzing a resulting compound to a compound of the formula I in which $R^2$ represents a metal cation, where appropriate liberating therefrom the free acid ($R^2$ = hydrogen), and where appropriate converting the free acid into compounds of the formula I with $R^2$ = alkyl or alkenyl, each having up to 8 carbon atoms, ammonium ion, benzyl or appropriately substituted benzyl,
d) and converting a resulting compound of the formula I where appropriate into a lactone of the formula II

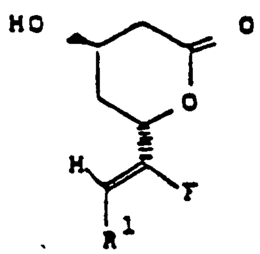

II

in which $R^1$ has the specified meaning.

**2.** A process for the preparation of a pharmaceutical product, which comprises converting a compound obtainable as claimed in claim 1 into a suitable administration form.

**3.** A compound of the formula III

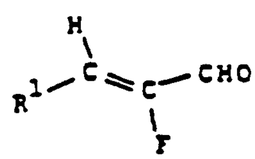

III

in which $R^1$ has the meaning specified for formula I in claim 1.

**4.** A compound of the formula IV

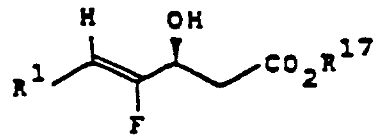

IV

in which $R^1$ has the meaning specified for formula I in claim 1, and $R^{17}$ is alkyl having 1-8 carbon atoms.

**5.** A compound of the formula V

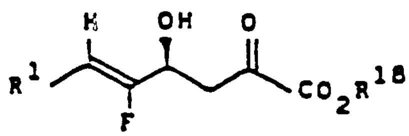

V

in which $R^1$ has the meaning specified for formula I in claim 1, and $R^{18}$ is alkyl having 1-8 carbon atoms.

**6.** A compound of the formula VI

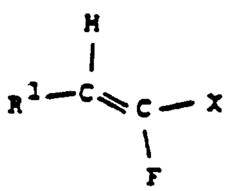

VI

in which $R^1$ has the meaning specified for formula I in claim 1, and X is a nitrile group (C≡N) or an ester group $COOR^{19}$, where $R^{19}$ is alkyl having 1-8 carbon atoms.

**7.** A compound of the formula VII

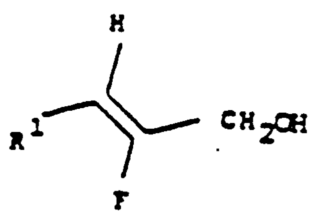

VII

in which $R^1$ has the meaning specified for formula I in claim 1.

**Revendications**

**Revendications pour les Etats contractants suivants : AT, BE, CH, DE, FR, GB, IT, LI, LU, NL, SE**

1. Acides 6-fluoro-3,5-dihydroxycarboxyliques et leurs dérivés, de formule générale I

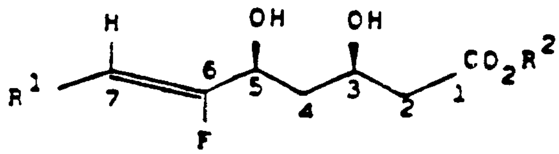

I

et lactones correspondantes de formule II

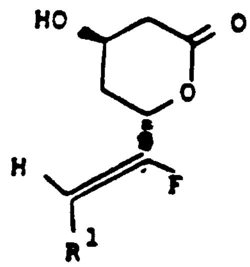

II

formules générales I et II dans lesquelles

$R^1$ représente le groupe des composés hétéroaromatiques à cycle à 6 chaînons, substitués, b

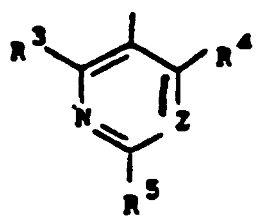

b

dans lesquels

Z représente un radical de formule -CH ou un atome d'azote,

$R^3$, $R^4$, $R^5$ représentent, indépendamment les uns des autres, un atome d'hydrogène ou un radical alkyle ou alcényle à chaîne droite ou ramifiée, ayant chacun jusqu'à 6 atomes de carbone, qui peut être éventuellement substitué sur l'atome de carbone terminal par un groupe cycloalkyle ou cycloalcényle ayant chacun de 3 à 6 atomes de carbone, un radical hydrocarboné cyclique saturé ou jusqu'à deux fois insaturé, ayant de 3 à 7 atomes de carbone, ou un radical aromatique choisi parmi les radicaux phényle, furyle, thiényle, pyridinyle, qui peut éventuellement porter sur le noyau 1-3 substituants identiques ou différents choisis parmi des atomes d'halogène et des groupes trifluorométhyle, alkyle ou alcényle ayant chacun jusqu'à 6 atomes de carbone, hydroxy, alcoxy ayant de 1 à 6 atomes de carbone, carboxy, carbalcoxy ayant de 1 à 6 atomes de carbone dans le fragment alcoxy, et

$R^2$ représente un atome d'hydrogène ou un radical alkyle ou alcényle à chaîne droite ou ramifiée, ayant jusqu'à 8 atomes de carbone, un radical benzyle dont le noyau peut être 1-2 fois substitué par un atome d'halogène ou par un groupe alkyle ayant de 1 à 4 atomes de carbone, un métal alcalin ou un ion ammonium $NR^{13}R^{14}R^{15}R^{16}$, $R^{13}$, $R^{14}$, $R^{15}$ et $R^{16}$ étant identiques ou différents et représentant un atome d'hydrogène ou un

groupe alkyle ayant de 1 à 4 atomes de carbone ou hydroxyalkyle ayant de 1 à 4 atomes de carbone.

**2.** Procédé pour la préparation des composés de formules générales I et II caractérisé en ce que
a) on convertit des aldéhydes convenablement substitués de formule III

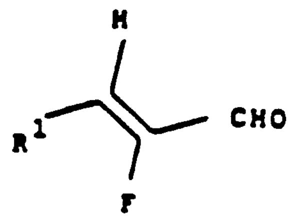

III

dans laquelle $R^1$ a la signification indiquée dans la revendication 1, en les hydroxyesters correspondants de formule générale IV

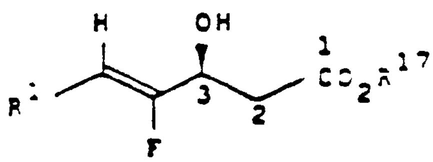

IV

dans laquelle $R^1$ a la signification indiquée dans la revendication 1 et $R^{17}$ représente un groupe protecteur d'acide optiquement actif approprié, qui détermine la configuration stéréochimique en C-3, ou un radical alkyle ayant de 1 à 8 atomes de carbone,
b) on convertit les composés optiquement actifs de formule IV, avec des énolates d'esters acétiques achiraux, soit directement, soit après conversion préliminaire en les esters alkyliques correspondants, en les composés optiquement actifs de formule V

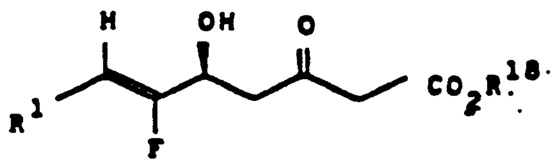

V

dans laquelle $R^1$ a la signification indiquée dans la revendication 1 et $R^{18}$ est un groupe alkyle ayant de 1 à 8 atomes de carbone,
c) on convertit les hydroxycétoesters de formule V en les composés 6-fluoro-3,5-dihydroxy correspondants de formule I

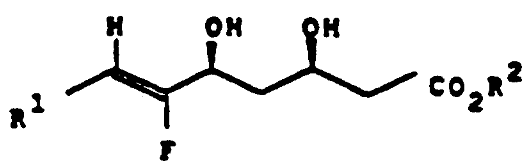

I

dans laquelle $R^1$ a la signification indiquée à propos de la formule I dans la revendication 1, et $R^2$ est un groupe alkyle ayant de 1 à 8 atomes de carbone, et éventuellement on saponifie un composé obtenu pour aboutir à un composé de formule I dans lequel $R^2$ représente un cation métallique, éventuellement on libère l'acide libre ($R^2$ = H) à partir de celui-ci, et on convertit éventuellement

l'acide libre en composés de formule I dans lesquels $R^2$ est un groupe alkyle ou alcényle ayant jusqu'à 8 atomes de carbone, un ion ammonium, le groupe benzyle ou un groupe benzyle convenablement substitué,

d) et éventuellement on convertit un composé de formule I obtenu en une lactone de formule II

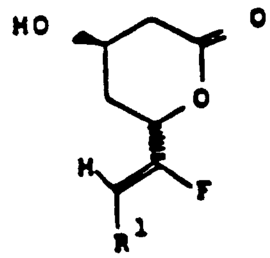

II

dans laquelle $R^1$ a la signification indiquée dans la revendication 1.

**3.** Composition pharmaceutique caractérisée en ce qu'elle contient un composé selon la revendication 1.

**4.** Composés selon la revendication 1, pour la prophylaxie et le traitement de l'artériosclérose et de l'hypercholestérolémie.

**5.** Composés de formule III

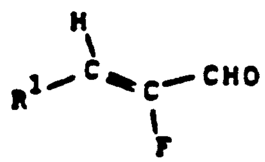

III

dans laquelle $R^1$ a la signification indiquée à propos de la formule I dans la revendication 1.

**6.** Composés de formule IV

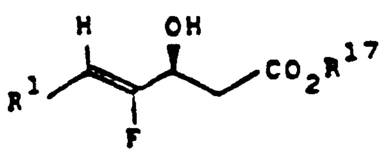

IV

dans laquelle $R^1$ a la signification indiquée à propos de la formule I dans la revendication 1 et $R^{17}$ est un groupe alkyle ayant de 1 à 8 atomes de carbone.

**7.** Composés de formule V

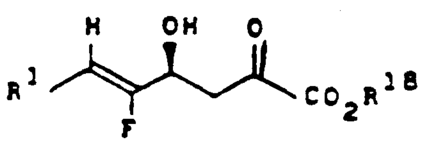

V

dans laquelle $R^1$ a la signification indiquée à propos de la formule I dans la revendication 1 et $R^{18}$ est un groupe alkyle ayant de 1 à 8 atomes de carbone.

**8.** Composés de formule VI

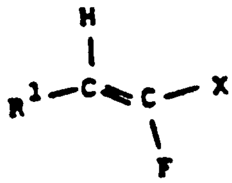

VI

dans laquelle $R^1$ a la signification indiquée à propos de la formule I dans la revendication 1 et X est un groupe nitrile (C≡N) ou un groupe ester $COOR^{19}$, $R^{19}$ étant un groupe alkyle ayant de 1 à 8 atomes de carbone.

**9.** Composés de formule VII

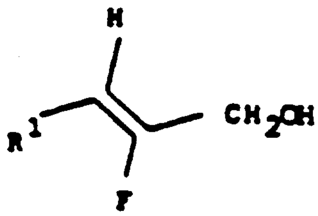

VII

dans laquelle $R^1$ a la signification indiquée à propos de la formule I dans la revendication 1.

**Revendications pour l'Etat contractant suivant : ES**

**1.** Procédé pour la préparation d'acides 6-fluoro-3,5-dihydroxycarboxyliques et de leurs dérivés, de formule générale I

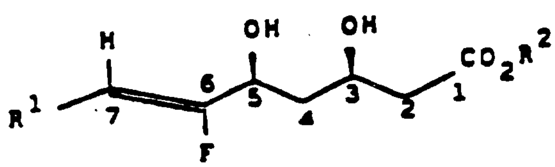

I

et des lactones correspondantes de formule II

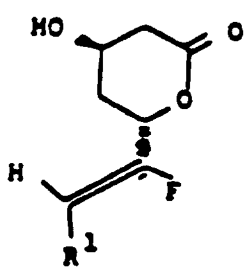

II

formules générales I et II dans lesquelles

$R^1$ représente le groupe des composés hétéroaromatiques à cycle à 6 chaînons, substitués, b

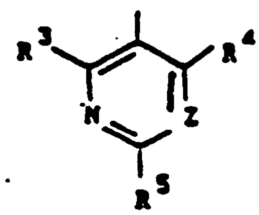

b

dans lesquels

Z représente un radical de formule -CH ou un atome d'azote,

$R^3$, $R^4$, $R^5$ représentent, indépendamment les uns des autres, un atome d'hydrogène ou un radical alkyle ou alcényle à chaîne droite ou ramifiée, ayant chacun jusqu'à 6 atomes de carbone, qui peut être éventuellement substitué sur l'atome de carbone terminal par un groupe cycloalkyle ou cycloalcényle ayant chacun de 3 à 6 atomes de carbone, un radical hydrocarboné cyclique saturé ou jusqu'à deux fois insaturé, ayant de 3 à 7 atomes de carbone, ou un radical aromatique choisi parmi les radicaux phényle, furyle, thiényle, pyridinyle, qui peut éventuellement porter sur le noyau 1-3 substituants identiques ou différents choisis parmi des atomes d'halogène et des groupes trifluorométhyle, alkyle ou alcényle ayant chacun jusqu'à 6 atomes de carbone, hydroxy, alcoxy ayant de 1 à 6 atomes de carbone, carboxy, carbalcoxy ayant de 1 à 6 atomes de carbone dans le fragment alcoxy, et

$R^2$ représente un atome d'hydrogène ou un radical alkyle ou alcényle à chaîne droite ou ramifiée, ayant jusqu'à 8 atomes de carbone, un radical benzyle dont le noyau peut être 1-2 fois substitué par un atome d'halogène ou par un groupe alkyle ayant de 1 à 4 atomes de carbone, un métal alcalin ou un ion ammonium $NR^{13}R^{14}R^{15}R^{16}$, $R^{13}$, $R^{14}$, $R^{15}$ et $R^{16}$ étant identiques ou différents et représentant un atome d'hydrogène ou un groupe alkyle ayant de 1 à 4 atomes de carbone ou hydroxyalkyle ayant de 1 à 4 atomes de carbone,

caractérisé en ce que

a) on convertit des aldéhydes convenablement substitués de formule III

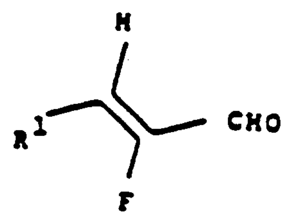

III

dans laquelle $R^1$ a la signification indiquée, en les hydroxyesters correspondants de formule générale IV

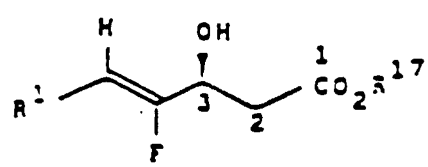

IV

dans laquelle $R^1$ a la signification indiquée et $R^{17}$ représente un groupe protecteur d'acide optiquement actif approprié, qui détermine la configuration stéréochimique en C-3, ou un radical alkyle ayant de 1 à 8 atomes de carbone,

b) on convertit les composés optiquement actifs de formule IV, avec des énolates d'esters acétiques achiraux, soit directement, soit après conversion préliminaire en les esters alkyliques correspondants, en les composés optiquement actifs de formule V

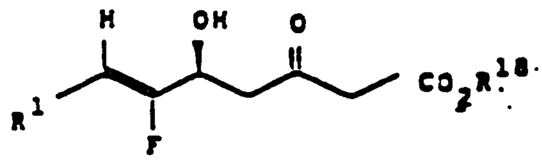

V

dans laquelle $R^1$ a la signification indiquée et $R^{18}$ est un groupe alkyle ayant de 1 à 8 atomes de carbone,

c) on convertit les hydroxycétoesters de formule V en les composés 6-fluoro-3,5-dihydroxy correspondants de formule I

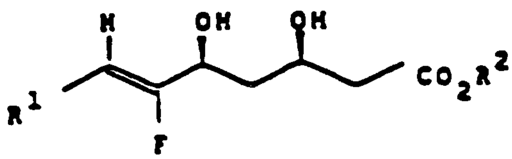

I

dans laquelle $R^1$ a la signification indiquée à propos de la formule I, et $R^2$ est un groupe alkyle ayant de 1 à 8 atomes de carbone, et éventuellement on saponifie un composé obtenu pour aboutir à un composé de formule I dans lequel $R^2$ représente un cation métallique, éventuellement on libère l'acide libre ($R^2$ = H) à partir de celui-ci, et on convertit éventuellement l'acide libre en composés de formule I dans lesquels $R^2$ est un groupe alkyle ou alcényle ayant jusqu'à 8 atomes de carbone, un ion ammonium, le groupe benzyle ou un groupe benzyle convenablement substitué,

d) et éventuellement on convertit un composé de formule I obtenu en une lactone de formule II

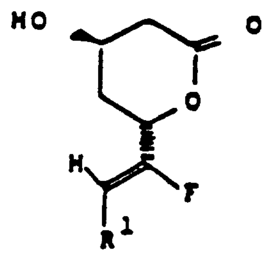

II

dans laquelle $R^1$ a la signification indiquée.

**2.** Procédé pour la préparation d'une composition pharmaceutique caractérisé en ce que l'on convertit en une forme d'administration appropriée un composé pouvant être obtenu selon la revendication 1.

**3.** Procédé selon la revendication 1, caractérisé en ce qu'on isole un composé de formule III

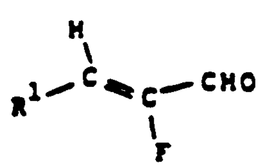

III

dans laquelle $R^1$ a la signification indiquée à propos de la formule I dans la revendication 1.

4. Procédé selon la revendication 1, caractérisé en ce qu'on isole un composé de formule IV

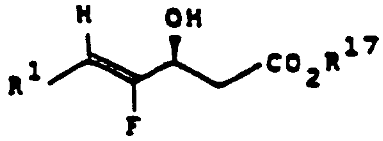

IV

dans laquelle $R^1$ a la signification indiquée à propos de la formule I dans la revendication 1 et $R^{17}$ est un groupe alkyle ayant de 1 à 8 atomes de carbone.

5. Procédé selon la revendication 1, caractérisé en ce qu'on isole un composé de formule V

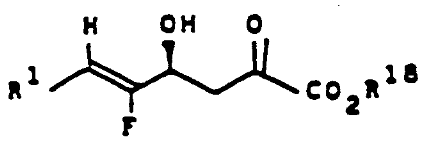

V

dans laquelle $R^1$ a la signification indiquée à propos de la formule I dans la revendication 1 et $R^{18}$ est un groupe alkyle ayant de 1 à 8 atomes de carbone.

6. Procédé selon la revendication 1, caractérisé en ce qu'on isole un composé de formule VI

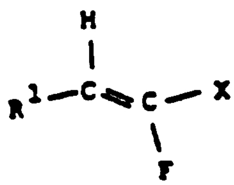

VI

dans laquelle $R^1$ a la signification indiquée à propos de la formule I dans la revendication 1 et X est un groupe nitrile (C≡N) ou un groupe ester $COOR^{19}$, $R^{19}$ étant un groupe alkyle ayant de 1 à 8 atomes de carbone.

7. Procédé selon la revendication 1, caractérisé en ce qu'on isole un composé de formule VII

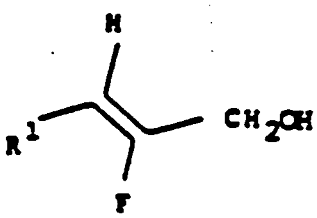

VII

dans laquelle $R^1$ a la signification indiquée à propos de la formule I dans la revendication 1.

**Revendications pour l'Etat contractant suivant : GR**

1. Procédé pour la préparation d'acides 6-fluoro-3,5-dihydroxycarboxyliques et de leurs dérivés, de formule générale I

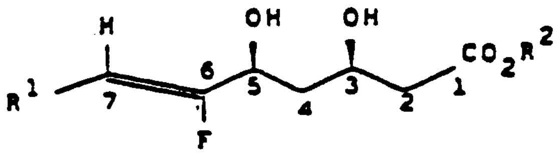

I

et des lactones correspondantes de formule II

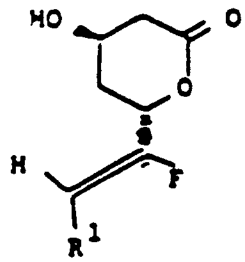

II

formules générales I et II dans lesquelles

$R^1$ représente le groupe des composés hétéroaromatiques à cycle à 6 chaînons, substitués, b

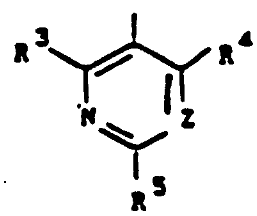

b

dans lesquels

Z représente un radical de formule -CH ou un atome d'azote,

$R^3$, $R^4$, $R^5$ représentent, indépendamment les uns des autres, un atome d'hydrogène ou un radical alkyle ou alcényle à chaîne droite ou ramifiée, ayant chacun jusqu'à 6 atomes de carbone, qui peut être éventuellement substitué sur l'atome de carbone terminal par un groupe cycloalkyle ou cycloalcényle ayant chacun de 3 à 6 atomes de carbone, un radical hydrocarboné cyclique saturé ou jusqu'à deux fois insaturé, ayant de 3 à 7 atomes de carbone, ou un radical aromatique choisi parmi les radicaux phényle, furyle, thiényle, pyridinyle, qui peut éventuellement porter sur le noyau 1-3 substituants identiques ou différents choisis parmi des atomes d'halogène et des groupes trifluorométhyle, alkyle ou alcényle ayant chacun jusqu'à 6 atomes de carbone, hydroxy, alcoxy ayant de 1 à 6 atomes de carbone, carboxy, carbalcoxy ayant de 1 à 6 atomes de carbone dans le fragment alcoxy, et

$R^2$ représente un atome d'hydrogène ou un radical alkyle ou alcényle à chaîne droite ou ramifiée, ayant jusqu'à 8 atomes de carbone, un radical benzyle dont le noyau peut être 1-2 fois substitué par un atome d'halogène ou par un groupe alkyle ayant de 1 à 4 atomes de carbone, un métal alcalin ou un ion ammonium $NR^{13}R^{14}R^{15}$ $R^{16}$, $R^{13}$, $R^{14}$, $R^{15}$ et $R^{16}$ étant identiques ou différents et représentant un atome d'hydrogène ou un groupe alkyle ayant de 1 à 4 atomes de carbone ou hydroxyalkyle ayant de 1 à 4 atomes de carbone,

caractérisé en ce que

a) on convertit des aldéhydes convenablement substitués de formule III

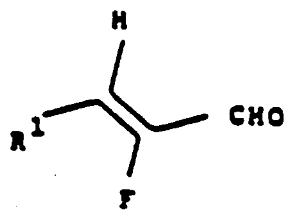

III

dans laquelle $R^1$ a la signification indiquée, en les hydroxyesters correspondants de formule générale IV

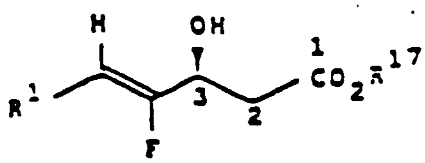

IV

dans laquelle $R^1$ a la signification indiquée et $R^{17}$ représente un groupe protecteur d'acide optiquement actif approprié, qui détermine la configuration stéréochimique en C-3, ou un radical alkyle ayant de 1 à 8 atomes de carbone,

b) on convertit les composés optiquement actifs de formule IV, avec des énolates d'esters acétiques achiraux, soit directement, soit après conversion préliminaire en les esters alkyliques correspondants, en les composés optiquement actifs de formule V

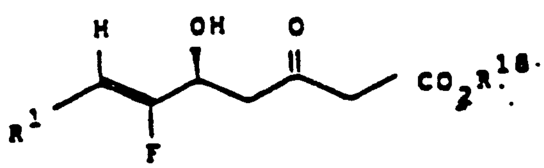

V

dans laquelle $R^1$ a la signification indiquée et $R^{18}$ est un groupe alkyle ayant de 1 à 8 atomes de carbone,

c) on convertit les hydroxycétoesters de formule V en les composés 6-fluoro-3,5-dihydroxy correspondants de formule I

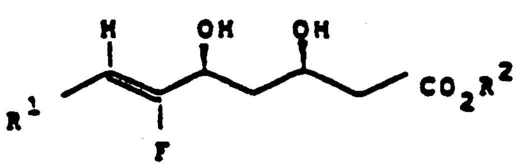

I

dans laquelle $R^1$ a la signification indiquée à propos de la formule I, et $R^2$ est un groupe alkyle ayant de 1 à 8 atomes de carbone, et éventuellement on saponifie un composé obtenu pour aboutir à un composé de formule I dans lequel $R^2$ représente un cation métallique, éventuellement on libère l'acide libre ($R^2$ = H) à partir de celui-ci, et on convertit éventuellement l'acide libre en composés de formule I dans lesquels $R^2$ est un groupe alkyle ou alcényle ayant jusqu'à 8 atomes de carbone, un ion ammonium, le groupe benzyle ou un groupe benzyle convenablement substitué,

d) et éventuellement on convertit un composé de formule I obtenu en une lactone de formule II

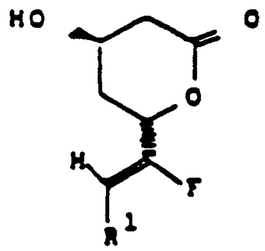

II

dans laquelle $R^1$ a la signification indiquée.

2. Procédé pour la préparation d'une composition pharmaceutique, caractérisé en ce que l'on convertit en une forme d'administration appropriée un composé pouvant être obtenu selon la revendication 1.

3. Composés de formule III

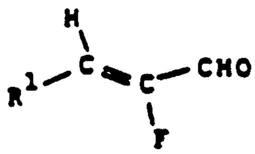

III

dans laquelle $R^1$ a la signification indiquée à propos de la formule I dans la revendication 1.

4. Composés de formule IV

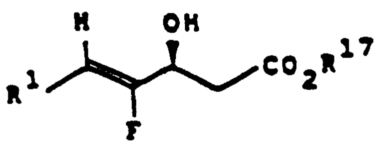

IV

dans laquelle $R^1$ a la signification indiquée à propos de la formule I dans la revendication 1 et $R^{17}$ est un groupe alkyle ayant de 1 à 8 atomes de carbone.

5. Composés de formule V

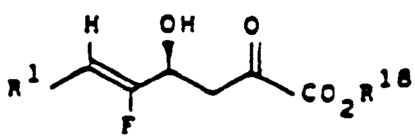

dans laquelle $R^1$ a la signification indiquée à propos de la formule I dans la revendication 1 et $R^{18}$ est un groupe alkyle ayant de 1 à 8 atomes de carbone.

**6.** Composés de formule VI

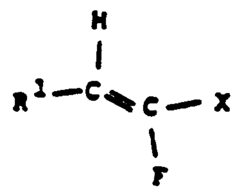

dans laquelle $R^1$ a la signification indiquée à propos de la formule I dans la revendication 1 et X est un groupe nitrile (C≡N) ou un groupe ester $COOR^{19}$, $R^{19}$ étant un groupe alkyle ayant de 1 à 8 atomes de carbone.

**7.** Composés de formule VII

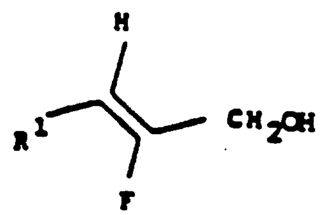

dans laquelle $R^1$ a la signification indiquée à propos de la formule I dans la revendication 1.